# EUROPÄISCHE PATENTSCHRIFT

(11) **EP 0 809 629 B1**
(45) Veröffentlichungstag und Bekanntmachung des Hinweises auf die Patenterteilung: **30.06.2004**
(21) Anmeldenummer: 96902951.1
(22) Anmeldetag: 31.01.1996
(51) Int. Cl.: C07D 207/40, C07D 209/96, C07D 401/12, C07D 307/94, A01N 43/38, A01N 43/12

(54) **2-PHENYLSUBSTITUIERTE HETEROCYCLISCHE 1,3-KETOENOLE ALS HERBIZIDE UND PESTIZIDE**
2-PHENYL-SUBSTITUTED HETEROCYCLIC 1,3-KETONOLS AS HERBICIDES AND PESTICIDES
1,3-CETOENOLS HETEROCYCLIQUES SUBSTITUES EN 2-PHENYLE UTILISES SOUS FORME D'HERBICIDES ET DE PESTICIDES

(30) Priorität: 13.02.1995 DE 19504621; 24.11.1995 DE 19543864
(43) Veröffentlichungstag der Anmeldung: 03.12.1997
(73) Patentinhaber: Bayer CropScience AG, 40789 Monheim (DE)
(72) Erfinder: FISCHER, Reiner, D-40789 Monheim (DE); BRETSCHNEIDER, Thomas, D-53797 Lohmar (DE); HAGEMANN, Hermann, D-51375 Leverkusen (DE); LIEB, Folker, D-51375 Leverkusen (DE); LUI, Norbert, D-51061 Köln (DE); RUTHER, Michael, D-40789 Monheim (DE); WIDDIG, Arno, D-51519 Odenthal (DE); ERDELEN, Christoph, D-42799 Leichlingen (DE); WACHENDORFF-NEUMANN, Ulrike, D-56566 Neuwied (DE); SANTEL, Hans-Joachim, D-51371 Leverkusen (DE); DOLLINGER, Markus, D-51381 Leverkusen (DE); DAHMEN, Peter, D-41470 Neuss (DE); MENCKE, Norbert, D-51381 Leverkusen (DE); TURBERG, Andreas, D-40699 Erkrath (DE)
(86) Internationale Anmeldenummer: PCT/EP1996/000382
(87) Internationale Veröffentlichungsnummer: WO 1996/025395

(56) Entgegenhaltungen:
- EP-A- 0 442 077
- EP-A- 0 456 063
- EP-A- 0 508 126
- EP-A- 0 521 334
- EP-A- 0 528 156
- EP-A- 0 613 885
- EP-A- 0 647 637
- US-A- 5 393 729

## Beschreibung

Die Erfindung betrifft neue phenylsubstituierte cyclische Ketoenole, mehrere Verfahren zu ihrer Herstellung und ihre Verwendung als Schädlingsbekämpfungsmittel und Herbizide.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger (Liebigs Ann. Chem. 1985, 1095) synthetisiert. Eine biologische Wirksamkeit dieser Verbindungen wurde nicht beschrieben.

In EP-A-0 262 399 und GB-A-2 266 888 werden ähnlich strukturierte Verbindungen (3-Aryl-pyrrolidin-2,4-dione) offenbart, von denen jedoch keine herbizide, insektizide oder akarizide Wirkung bekannt geworden ist. Bekannt mit herbizider, insektizider oder akarizider Wirkung sind unsubstituierte, bicyclische 3-Aryl-pyrrolidin-2,4-dion-Derivate (EP-A-355 599 und EP-415 211) sowie substituierte monocyclische 3-Aryl-pyrrolidin-2,4-dion-Derivate (EP-A-377 893 und EP-442 077).

Weiterhin bekannt sind polycyclische 3-Arylpyrrolidin-2,4-dion-Derivate (EP-442 073) sowie 1H-Arylpyrrolidin-dion-Derivate (EP-456 063, EP-521 334, EP-596 298, EP-613 884, EP-613 885, WO 94/01 997 und WO 95/01358).

Es ist bekannt, daß bestimmte substituierte Δ³-Dihydrofuran-2-on-Derivate herbizide Eigenschaften besitzen (vgl. DE-A-4 014 420). Die Synthese der als Ausgangsverbindungen verwendeten Tetronsäurederivate (wie z.B. 3-(2-Methyl-phenyl)-4-hydroxy-5-(4-fluorphenyl)-Δ³-dihydrofuranon-(2)) ist ebenfalls in DE-A-4 014 420 beschrieben. Ähnlich strukturierte Verbindungen ohne Angabe einer insektiziden und/oder akariziden Wirksamkeit sind aus der Publikation Campbell et al., J. Chem. Soc., Perkin Trans. 1, 1985, (8) 1567-76 bekannt. Weiterhin sind 3-Aryl-Δ³-dihydrofuranon-Derivate mit herbiziden, akariziden und insektiziden Eigenschaften aus EP-528 156 und EP 647 637bekannt, jedoch ist die dort beschriebene Wirkung nicht immer ausreichend.

Aus der Literatur sind ferner bestimmte 3H-Pyrazol-3-on-Derivate, wie beispielsweise 1,2-Diethyl-1,2-dihydro-5-hydroxy-4-phenyl-3H-pyrazol-3-on oder {[5-Oxo-1,2-diphenyl-4-(p-sulfophenyl)-3-pyrazolin-3-yl]-oxy}-dinatriumsalz oder p-(3-Hydroxy-5-oxo-1,2-diphenyl-3-pyrazolin-4-yl)-benzolsulfonsäure bekannt (vgl. J. Heterocycl. Chem., 25(5), 1301-1305, 1988 oder J. Heterocycl. Chem., 25(5), 1307-1310, 1988 oder Zh. Obshch. Khim., 34(7), 2397-2402, 1964). Eine biologische Wirkung dieser Verbindungen wird aber nicht beschrieben.

Weiterhin ist bekannt, daß das Trinatriumsalz der 4,4',4"-(5-Hydroxy-3-oxo-1Hpyrazol-1,2,4(3H)-triyl)-tris-benzolsulfonsäure pharmakologische Eigenschaften besitzt (vgl. Farmakol. Toksikol. (Moscow), 38(2), 180-186, 1976). Seine Verwendung im Pflanzenschutz ist aber nicht bekannt.

Außerdem sind in EP-508 126 und in WO 92/16 510 4-Arylpyrazolidin-3,5-dion-Derivate mit herbiziden, akariziden und insektiziden Eigenschaften beschrieben.

Bestimmte, im Phenylring unsubstituierte Phenyl-pyron-Derivate sind bereits bekannt geworden (vgl. A.M. Chirazi, T. Kappe und E. Ziegler, Arch. Pharm. 309, 558 (1976) und K.-H. Boltze und K. Heidenbluth, Chem. Ber. 91, 2849), wobei für diese Verbindungen eine mögliche Verwendbarkeit als Schädlingsbekämpfungsmittel nicht angegeben wird. Im Phenylring substituierte Phenyl-pyron-Derivate mit herbiziden, akariziden und insektiziden Eigenschaften sind in EP-588 137 beschrieben.

Bestimmte, im Phenylring unsubstituierte 5-Phenyl-1,3-thiazin-Derivate sind bereits bekannt geworden (vgl. E. Ziegler und E. Steiner, Monatsh. 95, 147 (1964), R. Ketcham, T. Kappe und E. Ziegler, J. Heterocycl. Chem. 10, 223 (1973)), wobei für diese Verbindungen eine mögliche Anwendung als Schädlingsbekämpfungsmittel nicht angegeben wird. Im Phenylring substituierte 5-Phenyl-1,3-thiazin-Derivate mit herbizider, akarizider und insektizider Wirkung sind in WO 94/14 785 beschrieben.

Die Wirksamkeit und Wirkungsbreite dieser Verbindungen ist jedoch insbesondere bei niedrigen Aufwandmengen und Konzentrationen nicht immer voll zufriedenstellend. Weiterhin ist die Pflanzenverträglichkeit dieser Verbindungen nicht immer ausreichend.

Es wurden nun neue Verbindungen der Formel (I) 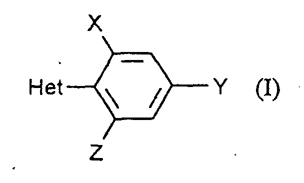 gefunden,
in welcher
- X: für Halogen, C₁-C₆-Alkyl, C₁-C₆-Halogenalkyl, C₁-C₆-Halogenalkoxy oder Cyano steht,
- Y: für C₁-C₆-Alkyl, C₁-C₆-Alkylsulfonyl, C₁-C₆-Halogenalkyl, C₁-C₆-Halogenalkoxy oder Cyano steht,
- Z: für C₁-C₆-Alkyl steht, wobei mindestens einer der Substituenten X und Y nicht für Alkyl oder Halogenalkyl steht,
- Het: für eine der Gruppen 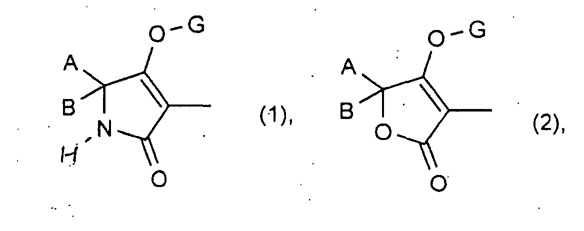 steht,
- A, B: und das Kohlenstoffatom, an das sie gebunden sind, für gesättigtes C₃-C₁₀-Cycloalkyl stehen, in welchem gegebenenfalls ein Ringglied gegebenenfalls einfach oder mehrfach durch C₁-C₈-Alkyl oder C₁-C₈-Alkoxy, substituiert ist,
- G: für Wasserstoff (a) oder für eine der Gruppen 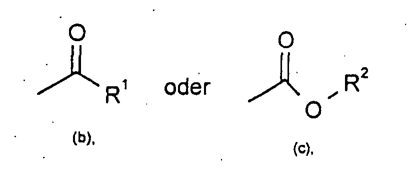 steht,
in welchen
R¹ für C₁-C₂₀-Alkyl oder C₁-C₈-Alkoxy-C₁-C₈-alkyl steht,
R² für C₁-C₂₀-Alkyl steht.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Unter Einbeziehung der Bedeutungen (1) bis (6) der Gruppe Het ergeben sich folgende hauptsächliche Strukturen (I-1) bis (I-6): 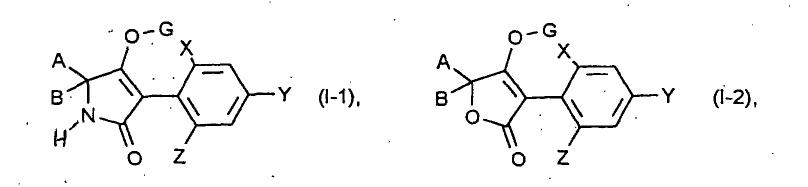 worin
A, B, G, X, Y und Z die oben angegebene Bedeutung haben.

Unter Einbeziehung der verschiedenen Bedeutungen (a), (b), (c), der Gruppe G ergeben sich folgende hauptsächliche Strukturen (I-1-a) bis (I-1-c), wenn Het für die Gruppe (1) steht, 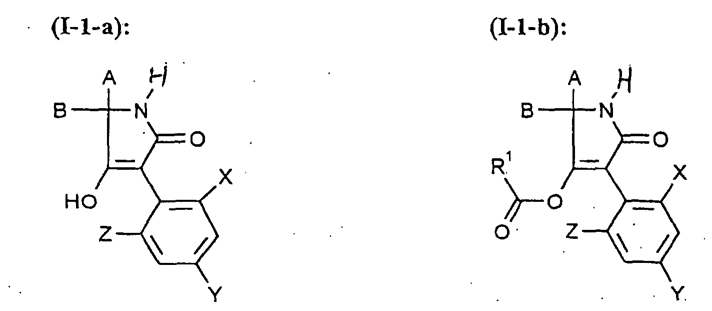 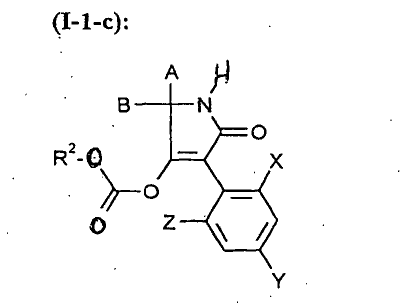 worin
A, B, X, Y, Z, R¹, R², und die oben angegebenen Bedeutungen besitzen.

Unter Einbeziehung der verschiedenen Bedeutungen (a), (b), (c), der Gruppe G ergeben sich folgende hauptsächliche Strukturen (I-2-a) bis (I-2-c), wenn Het für die Gruppe (2) steht, 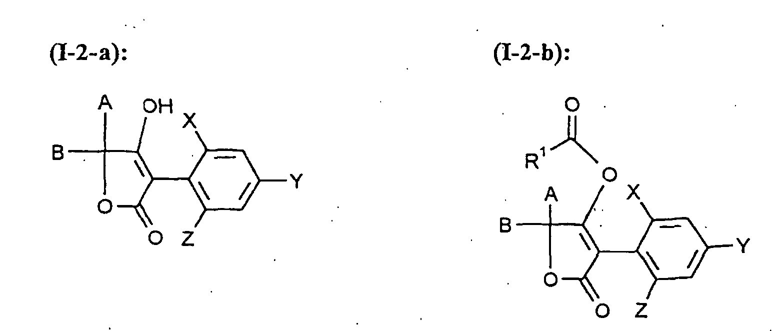 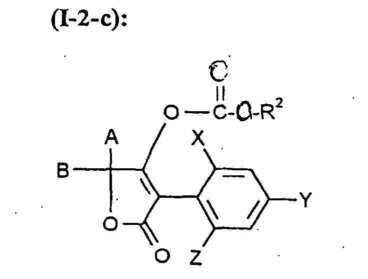 worin
A, B, X, Y, Z, R¹, R², die oben angegebene Bedeutung haben.

Weiterhin wurde gefunden, daß man die neuen Verbindungen der Formel (I) nach einem der im folgenden beschriebenen Verfahren erhält:
(A) Man erhält substituierte 3-Phenylpyrrolidin-2,4-dione bzw. deren Ende der Formel (I-1-a) 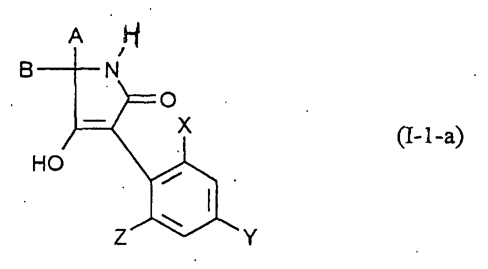 in welcher
   A, B, X, Y und Z die oben angegebenen Bedeutungen haben,
   wenn man
   N-Acylaminosäureester der Formel (II) 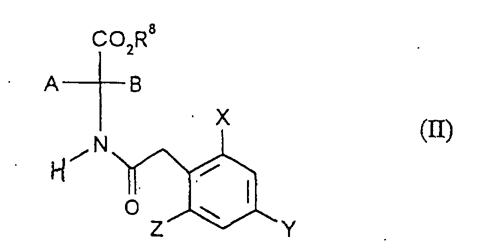 in welcher
   A, B, X, Y und Z die oben angegebenen Bedeutungen haben,
   und
   R⁸ für Alkyl (bevorzugt C₁-C₆-Alkyl) steht,
   in Gegenwart eines Verdünnungsmittels und in Gegenwart einer Base intramolekular kondensiert.
(B) Außerdem wurde gefunden, daß man substituierte 3-Phenyl-4-hydroxy-Δ³dihydrofuranon-Derivate der Formel (I-2-a) 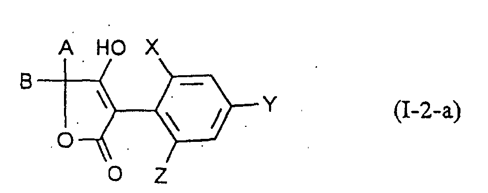 in welcher
   A, B, X, Y und Z die oben angegebenen Bedeutungen haben,
   erhält, wenn man
   Carbonsäureester der Formel (III) 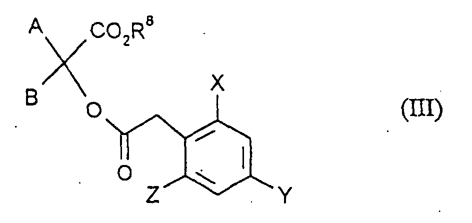 in welcher
   A, B, X, Y, Z und R⁸ die oben angegebenen Bedeutungen haben,
   in Gegenwart eines Verdünnungsmittels und in Gegenwart einer Base intramolekular kondensiert.

Außerdem wurde gefunden,
(G) daß man die Verbindungen der oben gezeigten Formeln (I-1-b) bis (I-2-b), in welchen A, B, R¹, X, Y und Z die oben angebenen Bedeutungen haben, erhält, wenn man Verbindungen der oben gezeigten Formeln (I-1-a) bis (I-2-a), in welchen A, B, X, Y und Z die oben angegebenen Bedeutungen haben,
   α) mit Säurechloriden der Formel (X) 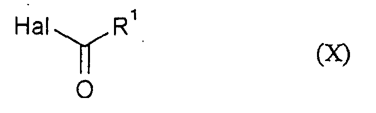 in welcher
      - R¹: die oben angegebene Bedeutung hat und
      - Hal: für Halogen (insbesondere Chlor oder Brom) steht
      oder
   β) mit Carbonsäureanhydriden der Formel (XI)

      R¹-CO-O-CO-R¹ (XI)

      in welcher
      - R¹: die oben angegebene Bedeutung hat,
      gegebenenfalls in Gegenwart eines Verdünnungsmittels und gegebenenfalls in Gegenwart eines Säurebindemittels umsetzt;
(H) daß man die Verbindungen der oben gezeigten Formeln (I-1-c) bis (I-2-c), in welchen A, B, R², X, Y und Z die oben angegebenen Bedeutungen haben, erhält, wenn man Verbindungen der oben gezeigten Formeln (I-1-a) bis (I-2-a), in welchen A, B, X, Y und Z die oben angegebenen Bedeutungen haben, jeweils
   mit Chlorameisensäureestern der Formel (XII)

   R²-O-CO-Cl (XII)

   in welcher
   - R²: die oben angegebenen Bedeutungen hat,
gegebenenfalls in Gegenwart eines Verdünnungsmittels und gegebenenfalls in Gegenwart eines Säurebindemittels umsetzt;

Die erfindungsgemäßen Verbindungen der Formeln (I-1-a), und (I-2-a), sind somit wichtige Zwischenprodukte für die Herstellung der erfindungsgemäßen Verbindungen der Formeln (I-1), und (I-2), in welchen G jeweils für eine der Gruppen b oder c), steht.

Weiterhin wurde gefunden, daß die neuen Verbindungen der Formel (I) eine sehr gute Wirksamkeit als Schädlingsbekämpfungsmittel, vorzugsweise als Insektizide, Akarizide und Herbizide aufweisen.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im folgenden erläutert:
- X: steht bevorzugt für Fluor, Chlor, Brom, C₁-C₄-Alkyl, C₁-C₄-Halogenalkyl, C₁-C₄-Halogenalkoxy oder Cyano,
- Y: steht bevorzugt für C₁-C₄-Alkyl, C₁-C₄-Alkylsulfonyl, C₁-C₄-Halogenalkyl, C₁-C₄-Halogenalkoxy oder Cyano,
- Z: steht bevorzugt für C₁-C₄-Alkyl steht, wobei mindestens einer der Substituenten X und Y nicht für Alkyl oder Halogenalkyl,
- Het: steht bevorzugt für eine der Gruppen 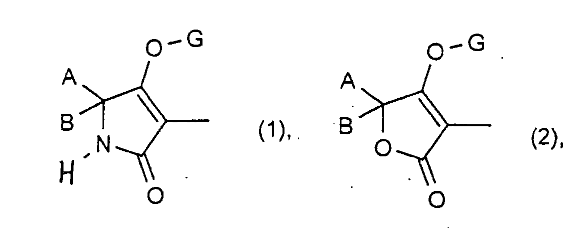
- A, B: und das Kohlenstoffatom an das sie gebunden sind, stehen bevorzugt für gesättigtes C₃-C₈-Cycloalkyl, in welchem gegebenenfalls ein Ringglied gegebenenfalls durch C₁-C₆-Alkyl, oder C₁-C₆-Alkoxy substituiert ist,
- G: steht bevorzugt für Wasserstoff (a) oder für eine der Gruppen 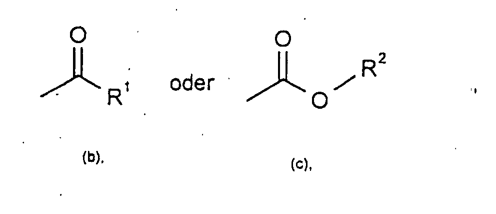
in welchen
- R¹: bevorzugt für C₁-C₁₆-Alkyl oder C₁-C₆-Alkoxy-C₁-C₆-alkyl steht,
- R²: bevorzugt für C₁-C₁₆-Alkyl steht,
- X: steht besonders bevorzugt für Fluor, Chlor, Brom, Methyl, Ethyl, Propyl, iso-Propyl, Trifluormethyl, Difluormethoxy, Trifluormethoxy, oder Cyano,
- Y: steht besonders bevorzugt für Methyl, Ethyl, n-Propyl, i-Propyl, n-Butyl, i-Butyl, tert.-Butyl, Trifluormethyl, Methylsulfonyl, Difluormethoxy, Trifluonnethoxy oder Cyano,
- Z: steht besonders bevorzugt für Methyl, Ethyl, n-Propyl, i-Propyl, n-Butyl, i-Butyl oder tert.-Butyl wobei mindestens einer der Substituenten X und Y nicht für Alkyl oder Halogenalkyl steht,
- Het: steht besonders bevorzugt für eine der Gruppen 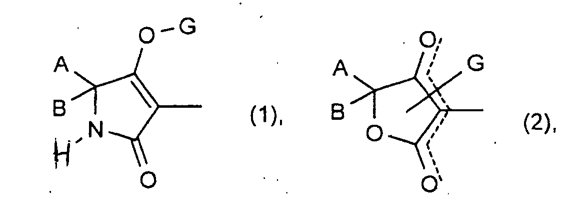
- A, B: und das Kohlenstoffatom an das sie gebunden sind stehen besonders bevorzugt, für gesättigtes C₃-C₈-Cycloalkyl, in welchem gegebenenfalls ein Ringglied gegebenenfalls durch Methyl, Ethyl, Propyl, Isopropyl, Butyl, iso-Butyl, sec.-Butyl, tert.-Butyl, Methoxy, Ethoxy, Propoxy, iso-Propoxy, Butoxy, iso-Butoxy, sek.-Butoxy oder tert.-Butoxy, substituiert ist, oder
G steht besonders bevorzugt für Wasserstoff (a) oder für eine der Gruppen 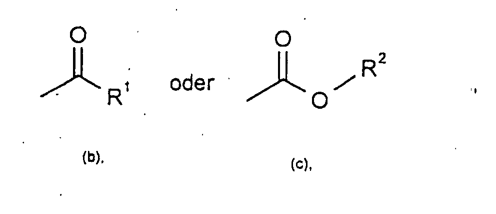 in welchen
R¹ besonders bevorzugt für C₁-C₁₄-Alkyl oder C₁-C₄-Alkoxy-C₁-C₆-alkyl steht,
R² besonders bevorzugt für C₁-C₁₄-Alkyl steht.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Im einzelnen seien außer den bei den Herstellungsbeispielen genannten Verbindungen die folgenden Verbindungen der Formel (I-1-a) genannt:

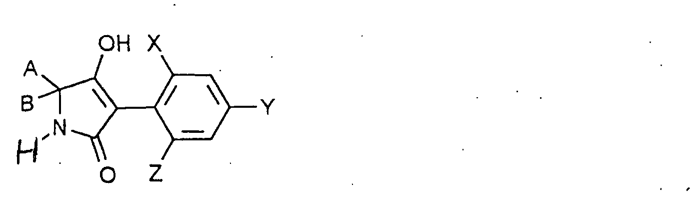

**Tabelle 1:**

| X = CH₃, Y = CN, Z = CH₃. | |
|---|---|
| A | B |
| -(CH₂)₂- | |
| -(CH₂)₄- | |
| -(CH₂)₅- | |
| -(CH₂)₆- | |
| -(CH₂)₇- | |
| -CH₂-CHCH₃-(CH₂)₃- | |
| -(CH₂)₂-CHCH₃-(CH₂)₂- | |
| -(CH₂)₂-CHC₂H₅-(CH₂)₂- | |
| -(CH₂)₂-CHC₃H₇-(CH₂)₂- | |
| -(CH₂)₂-CHi-C₃H₇-(CH₂)₂- | |
| -(CH₂)₂-CHOCH₃-(CH₂)₂- | |
| -(CH₂)₂-CHOC₂H₅-(CH₂)₂- | |
| -(CH₂)₂-CHOC₃H₇-(CH₂)₂- | |
| -(CH₂)₂-CHi-C₃H₇-(CH₂)₂- | |
| -(CH₂)₂-C(CH₃)₂-(CH₂)₂- | |
| -CH₂-(CHCH₃)₂-(CH₂)₂- | |
| -(CH₂)₃- | |
| -(CH₂)₄- | |
| -CH₂-CHCH₃-CH₂- | |
| -CH₂-CH₂-CHCH₃- | |
| -CH₂-CHCH₃-CHCH₃- | |

### Tabelle 2: A, B und D wie in Tabelle 1 angegeben

X = C₂H₅; Y = CN; Z = CH₃

### Tabelle 3: A, B und D wie in Tabelle 1 angegeben

X = C₂H₅; Y = CN; Z = C₂H₅

### Tabelle 4: A, B und D wie in Tabelle 1 angegeben

X = CN; Y = CH₃; Z = CH₃

### Tabelle 5: A, B und D wie in Tabelle 1 angegeben

X = CN; Y = C₂H₅; Z = CH₃

### Tabelle 6: A, B und D wie in Tabelle 1 angegeben

X = CN; Y = C₂H₅; Z = C₂H₅

### Tabelle 7: A, B und D wie in Tabelle 1 angegeben

X = CN; Y = CH₃; Z = C₂H₅

### Tabelle 12: A, B und D wie in Tabelle 1 angegeben

X = OCHF₂; Y = CH₃; Z = CH₃

### Tabelle 13: A, B und D wie in Tabelle 1 angegeben

X = O-CH₂CF₃; Y = CH₃; Z = CH₃

Im einzelnen seien außer den bei den Herstellungsbeispielen genannten Verbindungen die folgenden Verbindungen der Formel (I-2-a) genannt:

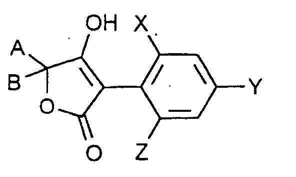

**Tabelle 16:**

| X = CH₃, Y = CN, Z = CH₃. | |
|---|---|
| A | B |
| -(CH₂)₂- | |
| -(CH₂)₄- | |
| -(CH₂)₅- | |
| -(CH₂)₆- | |
| -(CH₂)₇- | |
| -CH₂-CHCH₃-(CH₂)₃- | |
| -(CH₂)₂-CHCH₃-(CH₂)₂- | |
| -(CH₂)₂-CHC₂H₅-(CH₂)₂- | |
| -(CH₂)₂-CHC₃H₇-(CH₂)₂- | |
| -(CH₂)₂-CHi-C₃H₇-(CH₂)₂- | |
| -(CH₂)₂-CHOCH₃-(CH₂)₂- | |
| -(CH₂)₂-CHOC₂H₅-(CH₂)₂- | |
| -(CH₂)₂-CHOC₃H₇-(CH₂)₂- | |
| -(CH₂)₂-CHi-C₃H₇-(CH₂)₂- | |
| -(CH₂)₂-C(CH₃)₂-(CH₂)₂- | |
| -CH₂-(CHCH₃)₂-(CH₂)₂- | |

### Tabelle 17: A und B wie in Tabelle 16 angegeben

X = C₂H₅; Y = CN; Z = CH₃

### Tabelle 18: A und B wie in Tabelle 16 angegeben

X = C₂H₅; Y = CN; Z = C₂H₅

### Tabelle 19: A und B wie in Tabelle 16 angegeben

X = CN; Y = CH₃; Z = CH₃

### Tabelle 20: A und B wie in Tabelle 16 angegeben

X = CN; Y = C₂H₅; Z = CH₃

### Tabelle 21: A und B wie in Tabelle 16 angegeben

X = CN; Y = C₂H₅; Z = C₂H₅

### Tabelle 22: A und B wie in Tabelle 16 angegeben

X = CN; Y = CH₃; Z = C₂H₅

### Tabelle 27: A und B wie in Tabelle 16 angegeben

X = OCHF₂; Y = CH₃; Z = CH₃

### Tabelle 28: A und B wie in Tabelle 16 angegeben

X = O-CH₂CF₃; Y = CH₃; Z = CH₃

Verwendet man gemäß Verfahren (A) N-[(2-Methylthio-4-methoxy)-phenylacetyl]-1-amino-4-ethyl-cyclohexan-carbonsäureethylester als Ausgangsstoff, so kann der Verlauf des erfindungsgemäßen Verfahrens durch folgendes Reaktionsschema wiedergegeben werden:

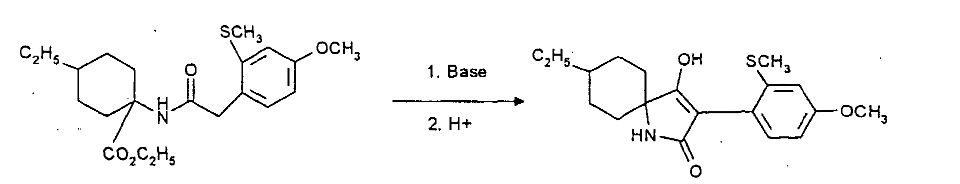

Verwendet man gemäß Verfahren (B) O-[(2-Chlor-4-sulfonylmethyl)-phenylacetyl]-hydroxyessigsäureethylester, so kann der Verlauf des erfindungsgemäßen Verfahrens durch folgendes Reaktionsschema wiedergegeben werden:

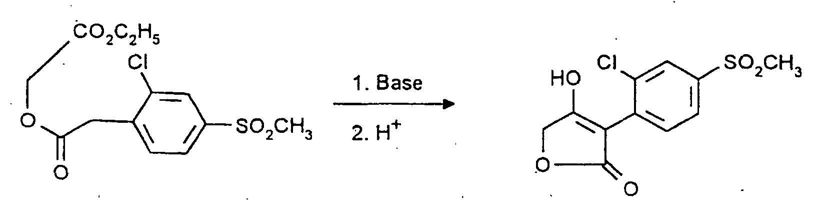

Verwendet man gemäß Verfahren (Gα) 3-[(2-Chlor-4-cyano)-phenyl]-5,5-dimethylpyrrolidin-2,4-dion und Pivaloylchlorid als Ausgangsstoffe, so kann der Verlauf des erfindungsgemäßen Verfahrens durch folgendes Reaktionsschema wiedergegeben werden:

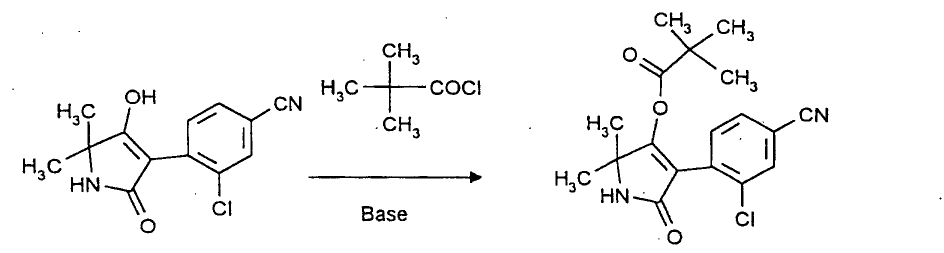

Verwendet man gemäß Verfahren (G) (Variante β) 3-[(4-Chlor-2-cyano)-phenyl]-4-hydroxy-5-phenyl-Δ³-dihydrofuran-2-on und Acetanhydrid als Ausgangsverbindungen, so kann der Verlauf des erfindungsgemäßen Verfahrens durch folgendes Reaktionsschema wiedergegeben werden:

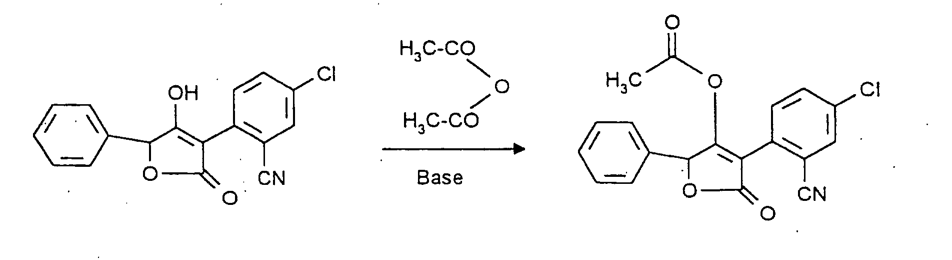

Verwendet man gemäß Verfahren (H) 8-[(2,4-Dicyano)-phenyl]-1,2-diaza-bicyclo-(4,3,0^{1,2})-nonan-7,9-dion und Chlorameisensäureethoxyethylester als Ausgangsverbindungen, so kann der Verlauf des erfindungsgemäßen Verfahrens durch folgendes Reaktionsschema wiedergegeben werden:

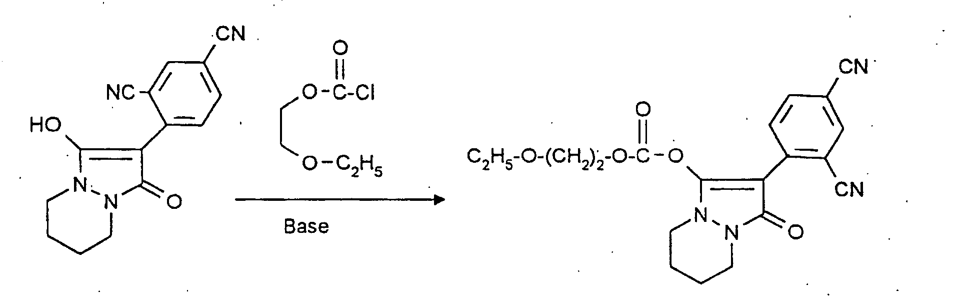

Die beim erfindungsgemäßen Verfahren (A) als Ausgangsstoffe benötigten Verbindungen der Formel (II) 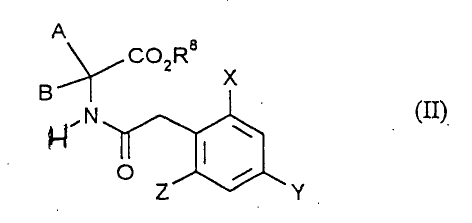 in welcher
A, B, X, Y, Z und R⁸ die oben angegebene Bedeutungen haben,
sind neu.

Man erhält die Acylaminosäureester der Formel (II) beispielsweise, wenn man Aminosäurederivate der Formel (XXI) 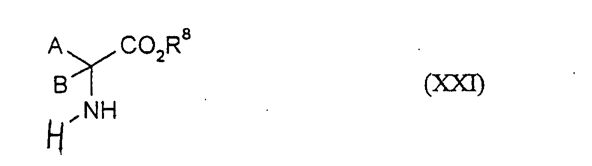 in welcher
- A, B, R⁸: die oben angegebenen Bedeutungen haben,
mit substituierten Phenylessigsäurehalogeniden der Formel (XXII) 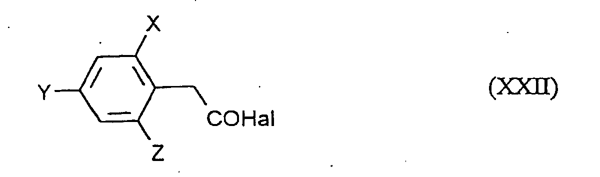 in welcher
- X, Y und Z: die oben angegebenen Bedeutungen haben und
- Hal: für Chlor oder Brom steht,
acyliert (Chem. Reviews 52, 237-416 (1953); Bhattacharya, Indian J. Chem. 6, 341-5, 1968)
oder wenn man Acylaminosäuren der Formel (XXIII) 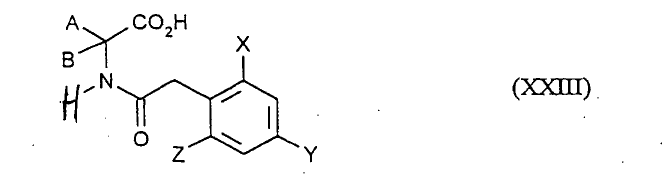 in welcher
- A, B, X, Y und Z: die oben angegebenen Bedeutungen haben,
verestert (Chem. Ind. (London) 1568 (1968)).

Die Verbindungen der Formel (XXIII) 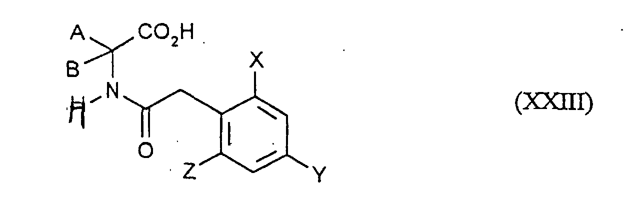 in welcher
- A, B, X, Y und Z: die oben angegebenen Bedeutungen haben,
sind neu.

Man erhält die Verbindungen der Formel (XXIII), wenn man Aminosäuren der Formel (XXIV)
in welcher 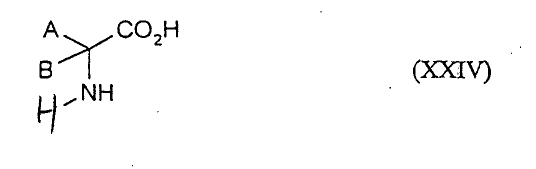
- A, B: die oben angegebenen Bedeutungen haben,
mit substituierten Phenylessigsäurehalogeniden der Formel (XXII) 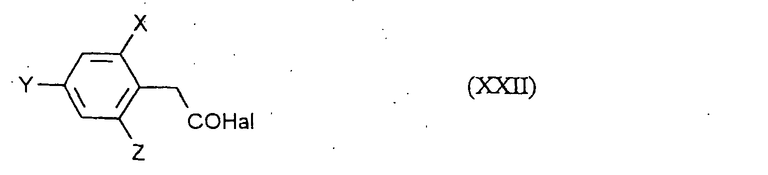 in welcher
- X, Y und Z: die oben angegebenen Bedeutungen haben und
- Hal: für Chlor oder Brom steht,
nach Schotten-Baumann acyliert (Organikum, VEB Deutscher Verlag der Wissenschaften, Berlin 1977, S. 505).

Die Verbindungen der Formel (XXII) sind teilweise bekannt oder lassen sich nach bekannten Verfahren darstellen (s. z.B. H. Henecka, Houben-Weyl, Methoden der Organischen Chemie, Bd. 8, S. 467-469 (1952)).

Verbindungen der Formel (XXII-a) 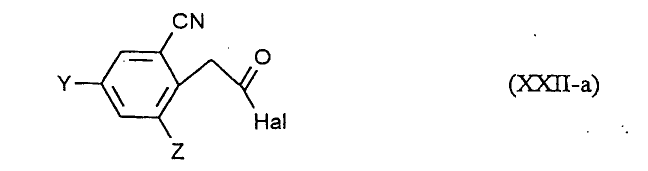 in welcher
- Hal: für Chlor oder Brom steht und
- Y und Z: die oben angegebenen Bedeutungen haben, aber nicht gleichzeitig für Wasserstoff stehen,
sind neu.

Man erhält die Verbindungen der Formel (XXII-a) beispielsweise, indem man substituierte Phenylessigsäuren der Formel (XXVIII-a) 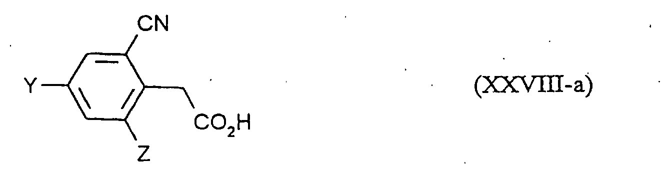 in welcher
- Y und Z: die oben angegebene Bedeutung haben,
mit Halogenierungsmitteln (z.B. Thionylchlorid, Thionylbromid, Oxalylchlorid, Phosgen, Phosphortrichlorid, Phosphortribromid oder Phosphorpentachlorid) gegebenenfalls in Gegenwart eines Verdünnungsmittels (z.B. gegebenenfalls chlorierten aliphatischen oder aromatischen Kohlenwasserstoffen wie Toluol oder Methylenchlorid) bei Temperaturen von -20°C bis 150°C, bevorzugt von -10°C bis 100°C, umsetzt.

Die Verbindungen der Formel (XXVIII-a) sind neu.

Man erhält die Verbindungen der Formel (XXVIII-a) beispielsweise, indem man substituierte Phenylessigsäureester der Formel (XXX-a) 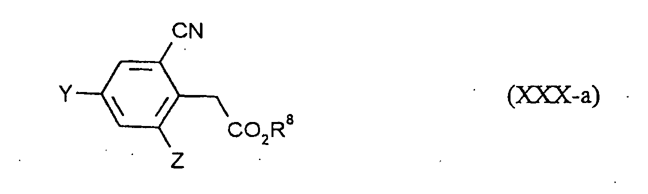 in welcher
- Y, Z und R⁸: die oben angegebene Bedeutung haben,
in Gegenwart einer Säure (z.B. einer anorganischen Säure wie Chlorwasserstoffsäure) oder einer Base (z.B. eines Alkalihydroxids wie Natrium- oder Kaliumhydroxid) und gegebenenfalls eines Verdünnungsmittels (z.B. eines wässrigen Alkohols wie Methanol oder Ethanol) bei Temperaturen zwischen 0°C und 150°C, bevorzugt zwischen 20°C und 100°C, hydrolysiert.

Die Verbindungen der Formel (XXX-a) sind neu.

Man erhält Verbindungen der Formel (XXX-a) 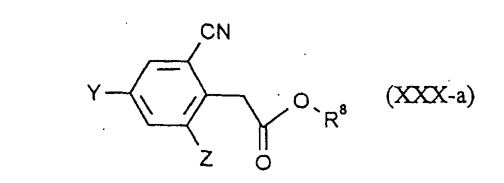 in welcher
- Y, Z und R⁸: die oben angegebene Bedeutung haben,
wenn man ortho-Halogenphenylessigsäureester der Formel (XXXIII-a) 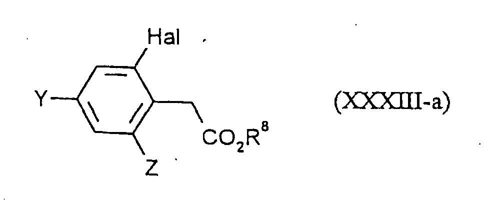 in welcher
- Y, Z und R⁸: die oben angegebene Bedeutung haben, und
- Hal: für Chlor, Brom, Iod, insbesondere für Brom steht,
mit Kupfercyanid in Gegenwart eines Verdünnungsmittels wie z.B. Dimethylformamid, Dimethylsulfoxid, N-Methylpyrrolidon oder Sulfolan bei Temperaturen von 50°C bis 250°C, vorzugsweise bei 80°C bis 200°C, umsetzt.

Die Verbindungen der Formel (XXXIII), in welcher Y und Z für Alkyl stehen, sind Gegenstand der deutschen Patentanmeldung der Anmelderin mit dem Aktenzeichen 19523850.8 vom 30.06.1995.

Verbindungen der Formel (XXII-b) 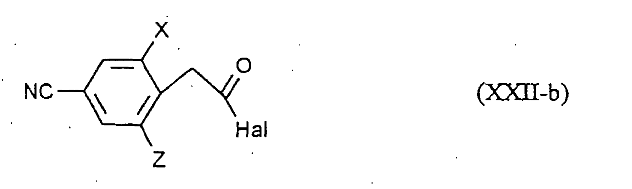 in welcher
- Hal: für Chlor oder Brom steht und
- X und Z: die oben angegebenen Bedeutungen haben,
sind neu.

Man erhält die Verbindungen der Formel (XXII-b) beispielsweise, indem man substituierte Phenylessigsäuren der Formel (XXVIII-b) 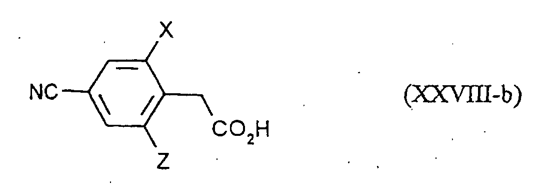 in welcher
- X und Z: die oben angegebene Bedeutung haben,
mit Halogenierungsmitteln (z.B. Thionylchlorid, Thionylbromid, Oxalylchlorid, Phosgen, Phosphortrichlorid, Phosphortribromid oder Phosphorpentachlorid) gegebenenfalls in Gegenwart eines Verdünnungsmittels (z.B. gegebenenfalls chlorierten aliphatischen oder aromatischen Kohlenwasserstoffen wie Toluol oder Methylenchlorid) bei Temperaturen von -20°C bis 150°C, bevorzugt von -10°C bis 100°C, umsetzt.

Die Verbindungen der Formel (XXVIII-b) sind neu.

Man erhält die Verbindungen der Formel (XXVIII-b) beispielsweise, indem man substituierte Phenylessigsäureester der Formel (XXX-b) 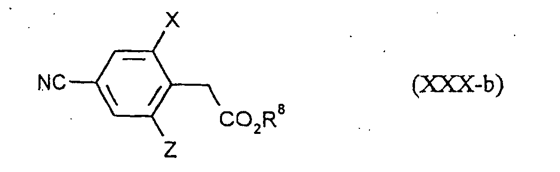 in welcher
- X, Z und R⁸: die oben angegebene Bedeutung haben,
in Gegenwart einer Säure (z.B. einer anorganischen Säure wie Chlorwasserstoffsäure) oder einer Base (z.B. eines Alkalihydroxids wie Natrium- oder Kaliumhydroxid) und gegebenenfalls eines Verdünnungsmittels (z.B. eines wässrigen Alkohols wie Methanol oder Ethanol) bei Temperaturen zwischen 0°C und 150°C, bevorzugt zwischen 20°C und 100°C, hydrolysiert.

Die Verbindungen der Formel (XXX-b) sind neu.

Man erhält Verbindungen der Formel (XXX-b) 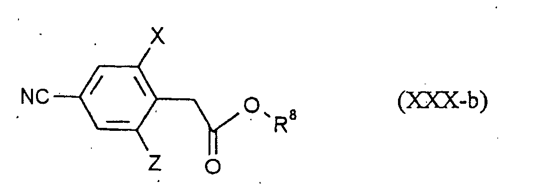 in welcher
- X, Z und R⁸: die oben angegebene Bedeutung haben,
wenn man para-Halogenphenylessigsäureester der Formel (XXXIII-b) 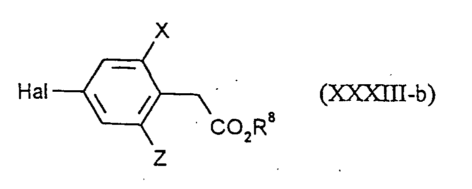 in welcher
- X, Z und R⁸: die oben angegebene Bedeutung haben, und
- Hal: für Chlor, Brom, Iod, insbesondere für Brom steht,
mit Kupfercyanid in Gegenwart eines Verdünnungsmittels wie z.B. Dimethylformamid, Dimethylsulfoxid, N-Methylpyrrolidon oder Sulfolan bei Temperaturen von 50°C bis 250°C, vorzugsweise bei 80°C bis 200°C, umsetzt.

Die Verbindungen der Formel (XXXIII-b), in welcher X und Z für Alkyl stehen, sind Gegenstand der deutschen Patentanmeldung der Anmelderin mit dem Aktenzeichen 19523850.8 vom 30.06.1995.

Verbindungen der Formel (XXII-c) 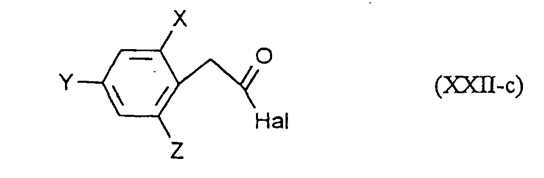 in welcher
- Hal: für Chlor oder Brom steht,
- X: für OCHF₂ oder OCH₂CF₃ steht und
- Y und Z: die oben angegebenen Bedeutungen haben, aber nicht gleichzeitig für Wasserstoff stehen,
sind neu.

Man erhält die Verbindungen der Formel (XXII-c) beispielsweise, indem man substituierte Phenylessigsäuren der Formel (XXVIII-c) 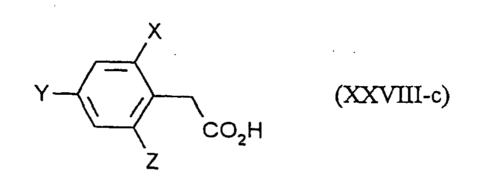 in welcher
- X: für OCHF₂ oder OCH₂CF₃ steht und
- Y und Z: die oben angegebene Bedeutung haben,
mit Halogenierungsmitteln (z.B. Thionylchlorid, Thionylbromid, Oxalylchlorid, Phosgen, Phosphortrichlorid, Phosphortribromid oder Phosphorpentachlorid) gegebenenfalls in Gegenwart eines Verdünnungsmittels (z.B. gegebenenfalls chlorierten aliphatischen oder aromatischen Kohlenwasserstoffen wie Toluol oder Methylenchlorid) bei Temperaturen von -20°C bis 150°C, bevorzugt von -10°C bis 100°C, umsetzt.

Die Verbindungen der Formel (XXVIII-c) sind neu.

Man erhält die Verbindungen der Formel (XXXIII-c) 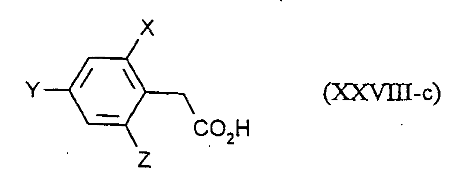 in welcher
- X: für OCHF₂ oder OCH₂CF₃ steht und
- Y und Z: die oben angegebene Bedeutung haben,
wenn man Phenylacetaldehyde der Formel (XXXIV-c) 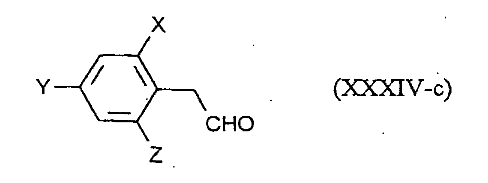 in welcher
- X: für OCHF₂ oder OCH₂CF₃ steht und
- Y und Z: die oben angegebene Bedeutung haben,
in Gegenwart eines Oxidationsmittels z.B. Natriumchromat, Natriumchlorit oder Sauerstoff in Gegenwart eines Katalysators, in Gegenwart eines Verdünnungsmittels und gegebenenfalls in Gegenwart eines Puffers bei -50°C bis 150°C, bevorzugt bei 0°C bis 100°C, umsetzt.

Die Verbindungen der Formel (XXXIV-c) sind neu.

Man erhält Phenylacetaldehyde der Formel (XXXIV-c) 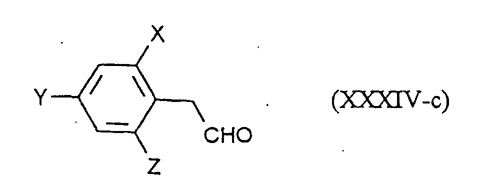 in welcher
- X: für OCHF₂ oder OCH₂CF₃ steht und
- Y und Z: die oben angegebene Bedeutung haben,
wenn man (2-Propen)-phenylether der Formel (XXXV-c) 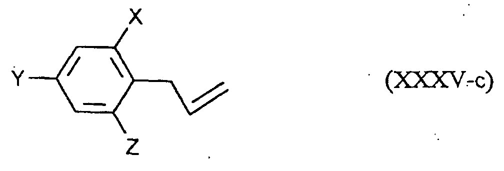 in welcher
- X: für OCHF₂ oder OCH₂CF₃ steht und
- Y und Z: die oben angegebene Bedeutung haben,
in Gegenwart von Ozon in einem Lösungsmittel z.B. Dichlormethan bei -120°C bis 0°C, bevorzugt bei -80°C bis -20°C, umsetzt und das entstandene Produkt mit einem Reduktionsmittel z.B. Dimethylsulfid reduziert.

Die Verbindungen der Formel (XXXV-c) sind neu, lassen sich aber nach im Prinzip bekannten Verfahren durch Frigenierung aus' den Phenolen in einfacher Weise, z.B. (Rico J., Wakselman, C., J. Fluorine Chem. 20 765-70 (1982)) oder durch nucleophile Substitution an Phenolaten darstellen, z.B. (Crossland, Wells, Shiner, J. AM. Chem. Soc. 93 4217 (1971)).

Die Verbindungen der Formel (XXI) und (XXTV) sind teilweise bekannt und/oder lassen sich nach bekannten Verfahren darstellen (siehe z.B. Compagnon, Miocque , Ann. Chim. (Paris) [14] 5, S. 11-22, 23-27 (1970)).

Die substituierten cyclischen Aminocarbonsäuren der Formel (XXIVa), in der A und B einen Ring bilden, sind im allgemeinen nach der Bucherer-Bergs-Synthese oder nach der Strecker-Synthese erhältlich und fallen dabei jeweils in unterschiedlichen Isomerenformen an. So erhält man nach den Bedingungen der Bucherer-Bergs-Synthese vorwiegend die Isomeren (im folgenden der Einfachheit halber als β bezeichnet), in welchen die Reste R und die Carboxylgruppe äquatorial stehen, während nach den Bedingungen der Strecker-Synthese vorwiegend die Isomeren (im folgenden der Einfachheit halber als α bezeichnet) anfallen, bei denen die Aminogruppe und die Reste R äquatorial stehen. 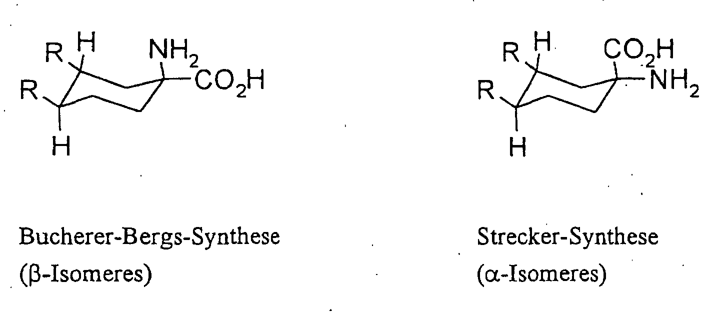 (L. Munday, J. Chem. Soc. 4372 (1961); J.T. Eward, C. Jitrangeri, Can. J. Chem. 53, 3339 (1975).

Weiterhin lassen sich die bei dem obigen Verfahren (A) verwendeten Ausgangsstoffe der Formel (II) 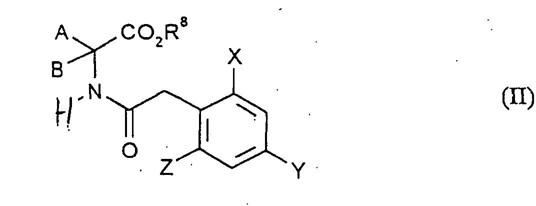 in welcher
- A, B, X, Y, Z und R⁸: die oben angegebenen Bedeutungen haben,
herstellen, wenn man Aminonitrile der Formel (XXV) 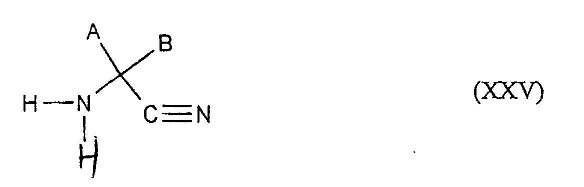 in welcher
- A, B: die oben angegebenen Bedeutungen haben,
mit substituierten Phenylessigsäurehalogeniden der Formel (XXII) 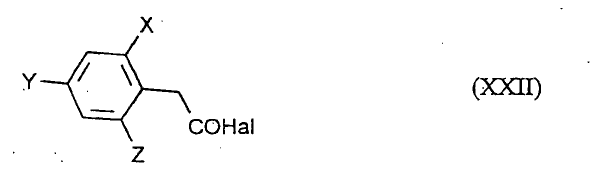 in welcher
- X, Y, Z und Hal: die oben angegebenen Bedeutungen haben,
zu Verbindungen der Formel (XXVI) 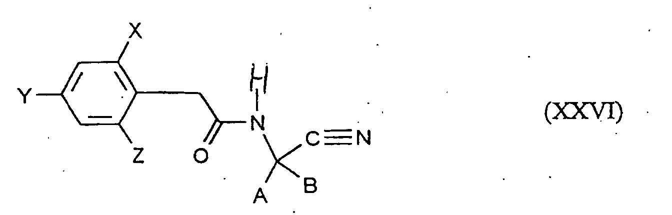 in welcher
- A, B, X, Y und Z: die oben angegebenen Bedeutungen haben,
umsetzt,
und diese anschließend einer sauren Alkoholyse unterwirft.

Die Verbindungen der Formel (XXVI) sind ebenfalls neu.

Die bei dem erfindungsgemäßen Verfahren (B) als Ausgangstoffe benötigten Verbindungen der Formel (III) 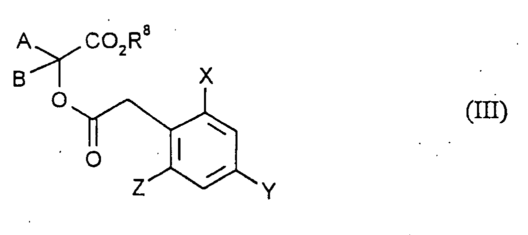 in welcher
- A, B, X, Y, Z und R⁸: die oben angegebenen Bedeutungen haben,
sind neu.

Sie lassen sich nach im Prinzip bekannten Methoden in einfacher Weise herstellen.

So erhält man O-Acyl-α-hydroxycarbonsäureester der Formel (III) beispielsweise, wenn man
2-Hydroxycarbonsäureester der Formel (XXVII) 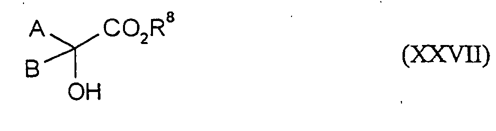 in welcher
- A, B und R⁸: die oben angegebenen Bedeutungen haben,
mit substituierten Phenylessigsäurehalogeniden der Formel (XXII) 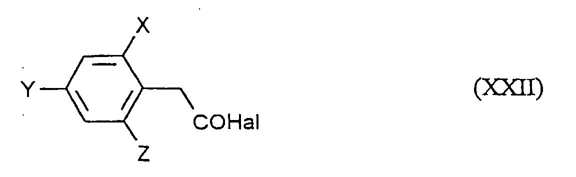 in welcher
- X, Y, Z und Hal: die oben angegebenen Bedeutungen haben,
acyliert (Chem. Reviews 52, 237-416 (1953)).

Weiterhin erhält man Verbindungen der Formel (III), wenn man
substituierte Phenylessigsäuren der Formel (XXVIII) 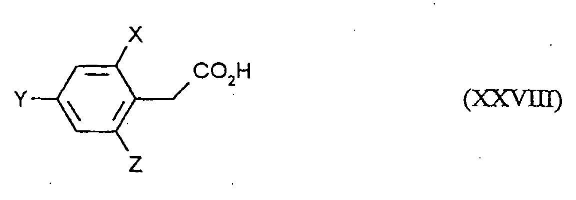 in welcher
- X, Y und Z: die oben angegebenen Bedeutungen haben,
mit α-Halogencarbonsäureestern der Formel (XXIX) 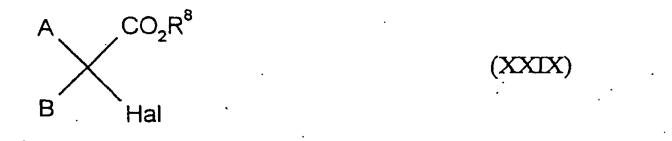 in welcher
- A, B und R⁸: die oben angegebenen Bedeutungen haben und
- Hal: für Chlor oder Brom steht,
alkyliert.

Die Verbindungen der Formel (XXVIII) sind teilweise bekannte Verbindungen und/oder lassen sich nach bekannten Verfahren in einfacher Weise herstellen, beispielsweise durch Verseifung von Phenylessigsäurenitrilen in Gegenwart von Säuren oder Basen oder durch Reduktion von Mandelsäureestern oder Phenylglyoxylsäuren mit Wasserstoff (vgl. Kindler et al., Chem. Ber. 76, 308, 1943). Die Verbindungen der Formel (XXIX) sind käuflich.

Die zur Durchführung der erfindungsgemäßen Verfahren (G), (H), (I), (J), (K), (L) und (M) außerdem als Ausgangsstoffe benötigten Säurehalogenide der Formel (X), Carbonsäureanhydride der Formel (XI), Chlorameisensäureester der Formel (XII), sind allgemein bekannte Verbindungen der organischen bzw. anorganischen Chemie.

Das Verfahren (A) ist dadurch gekennzeichnet, daß man Verbindungen der Formel (II), in welcher A, B, X, Y, Z und R⁸ die oben angegebenen Bedeutungen haben, in Gegenwart einer Base einer intramolekularen Kondensation unterwirft.

Als Verdünnungsmittel können bei dem erfindungsgemäßen Verfahren (A) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und - carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Die Reaktionstemperaturen können bei der Durchführung des erfindungsgemäßen Verfahrens (A) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponenten der Formel (II) und die deprotonierenden Basen im allgemeinen in etwa äquimolaren bis doppeltäquimolaren Mengen ein. Es ist jedoch auch möglich, die eine oder andere Komponente in einem größeren Überschuß (bis zu 3 Mol) zu verwenden.

Das Verfahren (B) ist dadurch gekennzeichnet, daß Verbindungen der Formel (III), in welcher A, B, X, Y, Z und R⁸ die oben angegebenen Bedeutungen haben, in Gegenwart eines Verdünnungsmittels und in Gegenwart einer Base einer intramolekularen Kondensation unterwirft.

Als Verdünnungsmittel können bei dem erfindungsgemäßen Verfahren (B) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, Iso-Propanol, Butanol, Iso-Butanol und tert.-Butanol eingesetzt werden.

Als Base (Deprotonierungsmittel) können bei der Durchführung des erfindungsgemäßen Verfahrens (B) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Die Reaktionstemperaturen können bei der Durchführung des erfindungsgemäßen Verfahrens (B) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (B) wird im allgemeinen unter Normaldruck durchgeführt.

Bei der Durchführung des erfindungsgemäßen Verfahrens (B) setzt man die Reaktionskomponenten der Formel (III) und die deprotonierenden Basen im allgemeinen in etwa äquimolaren Mengen ein. Es ist jedoch auch möglich, die eine oder andere Komponente in einem größeren Überschuß (bis zu 3 Mol) zu verwenden.

Das Verfahren (Gα) ist dadurch gekennzeichnet, daß man Verbindungen der Formeln (I-1-a) bis (I-2-a) jeweils mit Carbonsäurehalogeniden der Formel (X) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (Gα) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, Nitrile wie Acetonitril, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Als Säurebindemittel kommen bei der Umsetzung nach dem erfindungsgemäßen Verfahren (Gα) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperaturen können bei dem erfindungsgemäßen Verfahren (Gα) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Gα) werden die Ausgangsstoffe der Formeln (I-1-a) bis (I-2-a) und das Carbonsäurehalogenid der Formel (X) im allgemeinen jeweils in angenähert äquivalenten Mengen verwendet. Es ist jedoch auch möglich, das Carbonsäurehalogenid in einem größeren Überschuß (bis zu 5 Mol) einzusetzen. Die Aufarbeitung erfolgt nach üblichen Methoden.

Das Verfahren (Gβ) ist dadurch gekennzeichnet, daß man Verbindungen der Formeln (I-1-a) bis (I-2-a) mit Carbonsäureanhydriden der Formel (XI) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (Gβ) vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäureanhydrid gleichzeitig als Verdünnungsmittel fungieren.

Als gegebenenfalls zugesetzte Säurebindemittel kommen beim Verfahren (Gβ) vorzugsweise diejenigen Säurebindemittel in Frage, die auch bei der Verwendung von Säurehalogeniden vorzugsweise in Betracht kommen.

Die Reaktionstemperaturen können bei dem erfindungsgemäßen Verfahren (Gβ) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Gβ) werden die Ausgangsstoffe der Formeln (I-1-a) bis (I-2-a) und das Carbonsäureanhydrid der Formel (XI) im allgemeinen in jeweils angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (H) ist dadurch gekennzeichnet, daß man Verbindungen der Formeln (I-1-a) bis (I-2-a) jeweils mit Chlorameisensäureestern oder Chlorameisensäurethiolestern der Formel (XII) gegebenenfalls in Gegenwart eines Verdünnungsmittels und gegebenenfalls in Gegenwart eines Säurebindemittels umsetzt.

Als Säurebindemittel kommen bei der Umsetzung nach dem erfindungsgemäßen Verfahren (H) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (H) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, Nitrile wie Acetonitril, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan.

Die Reaktionstemperaturen können bei der Durchführung des erfindungsgemäßen Verfahrens (H) innerhalb eines größeren Bereiches variiert werden. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (H) wird im allgemeinen unter Normaldruck durchgeführt.

Bei der Durchführung des erfindungsgemäßen Verfahrens (H) werden die Ausgangsstoffe der Formeln (I-1-a) bis (I-2-a) und der entsprechende Chlorameisensäureester der Formel (XII) im allgemeinen jeweils in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt nach üblichen Methoden. Im allgemeinen geht man so vor, daß man ausgefallene Salze entfernt und das verbleibende Reaktionsgemisch durch Abziehen des Verdünnungsmittels einengt.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp..
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzüs spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe zeichnen sich durch eine hohe insektizide und akarizide Wirksamkeit aus.

Sie lassen sich mit besonders gutem Erfolg zur Bekämpfung von pflanzenschädigenden Insekten, wie beispielsweise gegen die Larven des Meerettichblattkäfers (Phaedon cochleariae) oder gegen die Larven der grünen Reiszikade (Nephotettix cincticeps) gegen die Raupen der Kohlschabe (Plutella maculipennis).

Die erfindungsgemäßen Wirkstoffe können weiterhin als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotola, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cycnodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Sachharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindunngen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst, Wein-, Citrus-, Nuß-, Bananen-, Kaffee-, Tee-, Gummi-, Ölpalm-, Kakao-, Beerenfruchtund Hopfenanlagen, auf Zier- und Sportrasen und Weideflächen und zur selektiven Unkrautbekämpfung in einjährigen Kulturen eingesetzt werden.

Die erfindungsgemäßen Wirkstoffe eignen sich sehr gut zur selektiven Bekämpfung monokotyler Unkräuter in dikotylen Kulturen im Vor- und Nachlaufverfahren. Sie können beispielsweise in Baumwolle oder Zuckerrüben mit sehr gutem Erfolg zur Bekämpfung von Schadgräser eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußsehalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

### Besonders günstige Mischpartner sind z.B. die folgenden:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino[alpha-(o-tolyloxy)-o-tolyl]acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyc-
lox,
Guazatine,
Hexachlorobenzol, Hexaconazol, Hymexazol,
Imazalil, Imibenconazol, Iminoctadin, Iprobenfos (IBP), Iprodion, Isoprothiolan, Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivemectin,
Lamda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram,
Omethoat, Oxamyl, Oxydemethon M, Oxydeprofos,
Parathion A, Parathion M, Permethrin, Phenthoat, Phorat, Phosalon, Phosmet, Phosphamdon, Phoxim, Pirimicarb, Pirimiphos M, Primiphos A, Profenofos, Promecarb, Propaphos, Propoxur, Prothiofos, Prothoat, Pymetrozin, Pyrachlophos, Pyradaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

### Herbizide:

beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxyalkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlörsulfuron, Cinosulfuron, Metsulfuron-methyl, Nicosulfuron, Primisulfuron, Pyrazosulfuronethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Der erfindungsgemäße Wirkstoff kann ferner in seinen handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygieneund Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..
Aus der Ordnung der Siphonapterida z.B. Pulex spp., Ctenocephalides spp., Xenopsylla spp., Ceratophyllus spp..
Aus der Ordnung der Heteropterida z.B. Cimex spp., Triatoma spp., Rhodnius spp., Panstrongylus spp..
Aus der Ordnung der Blattarida z.B. Blatta orientalis, Periplaneta americana, Blattela germanica, Supella spp..
Aus der Unterklasse der Acaria (Acarida) und den Ordnungen der Meta- sowie Mesostigmata z.B. Argas spp., Ornithodorus spp., Otabius spp., Ixodes spp., Amblyomma spp., Boophilus spp., Dermacentor spp., Haemaphysalis spp., Hyalomma spp., Rhipicephalus spp., Dermanyssus spp., Raillietia spp., Pneumonyssus spp., Sternostoma spp., Varroa spp..
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Beispielsweise zeigen sie eine hervorragende Wirksamkeit gegen Boophilus microplus.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändem, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen der Formel 1 eine hohe insektizide Wirkung gegen Insekten zeigen, die technische Materialien zerstören.

Beispielhaft und vorzugsweise - ohne jedoch zu limitieren - seien die folgenden Insekten genannt:

Käfer wie
Hylotrupes bajulus, Chlorophorus pilosis, Anobium punctatum, Xestobium rufovillosum, Ptilinus pecticomis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.

Hautflügler wie
Sirex juvencus, Urocerus gigas, Urocerus gigas taignus, Urocerus augur.

Termiten wie
Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

Borstenschwänze
wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen,

Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise α-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner seien Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron,
sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, genannt.

Die Herstellung und die Verwendung der erfindungsgemäßen Wirkstoffe gehen aus den nachfolgenden Beispielen hervor.

### Herstellungsbeispiele

### Beispiel (I-1-a-1)

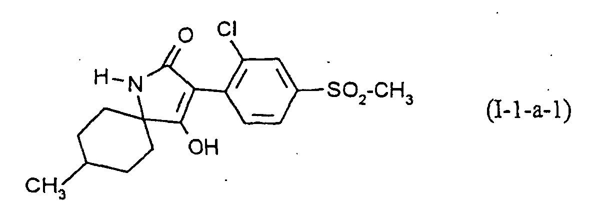

3,2 g (0,028 Mol) Kalium-tert.-butylat werden in 10 ml abs. Tetrahydrofuran (THF) vorgelegt. Bei Rückflußtemperatur tropft man 5,1 g (0,0127 Mol) der Verbindung gemäß Beispiel (II-1) in 30 ml abs. Toluol zu und rührt noch 1,5 Stunden unter Rückfluß. Dann gibt man 20 ml Wasser zu, trennt die Phasen, extrahiert die Toluolphase mit 10 ml Wasser und wäscht die vereinigten Wasserphasen mit Toluol. Die wäßrige Phase wird bei 15°C bis 20°C mit ca. 2,3 ml konz. Salzsäure angesauert. Der Niederschlag wird abgesaugt, gewaschen, getrocknet und aus Methyl-tert.-butylether (MTB-Ether)/n-Hexan umkristallisiert.
Ausbeute: 4,4 g (93 % d.Th.), Fp. 222°C.

Analog bzw. gemäß den allgemeinen Angaben zur Herstellung wurden die in der folgenden Tabelle aufgeführten Verbindungen der Formel (I-1-a) hergestellt:

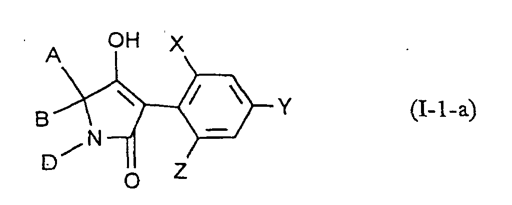

| Bsp.- Nr. | X | Y | Z | A | B | D | Fp.°C | Isomer |
|---|---|---|---|---|---|---|---|---|
| I-1-a-2 | CH₃ | CN | CH₃ | CH₃ | CH₃ | H | >220 | - |
| I-1-a-3 | CN | CH₃ | CH₃ | CH₃ | CH₃ | H | >220 | - |
| I-1-a-4 | CN | CH₃ | CH₃ | -(CH₂)₂-CHCH₃-(CH₂)₂- | | H | >220 | β |
| I-1-a-5 | C₂H₅ | CN | CH₃ | -(CH₂)₂-CHCH₃-(CH₂)₂- | | H | >220 | β |
| I-1-a-6 | CH₃ | CN | CH₃ | -(CH₂)₂-CHCH₃-(CH₂)₂- | | H | >220 | β |
| I-1-a-7 | C₂H₅ | CN | C₂H₅ | -(CH₂)₂-CHCH₃-(CH₂)₂- | | H | >220 | β |
| I-1-a-8 | OCH₂CF₃ | CH₃ | CH₃ | -(CH₂)₂-CHCH₃-(CH₂)₂- | | H | >220 | β |

### Beispiel (I-1-b-1)

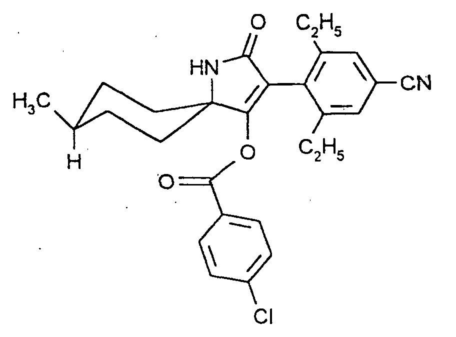

2,03 g der Verbindung gemäß Beispiel (I-1-a-7) und 8,5 ml Triethylamin in 50 ml Methylenchlorid werden bei 0°C bis 10°C mit 0,8 ml 4-Chlorbenzoylchlorid in 5 ml Methylenchlorid versetzt und bis zum Reaktionsende (DC-Kontrolle) bei Raumtemperatur gerührt. Man wäscht 2 mal mit 30 ml 0,5 N NaOH, trocknet und engt ein. Der Rückstand wird aus MTB-Ether/n-Hexan umkristallisiert.
Man erhält 1,6 g (55 % der Theorie) vom Fp.: >220°C.

Analog bzw. gemäß den allgemeinen Angaben zur Herstellung erhält man die folgenden Verbindungen der Formel (I-1-b):

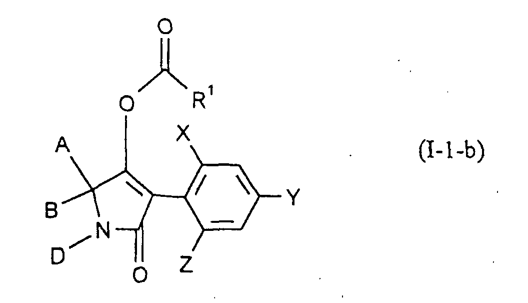

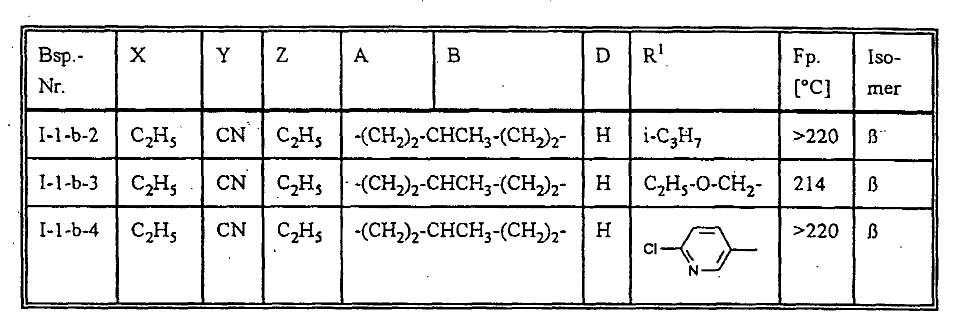

### Beispiel (I-1-c-1)

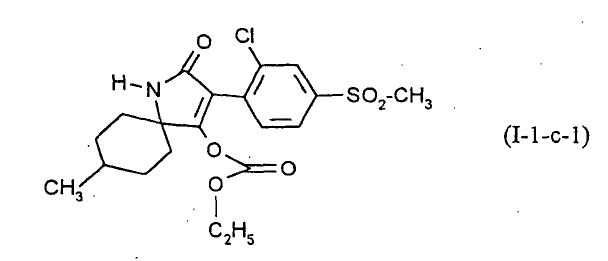

3,33 g (0,009 Mol) der Verbindung gemäß Beispiel (I-1-a-1) in 50 ml abs. Methylenchlorid werden zunächst mit 1,3 ml Triethylamin und dann bei 0°C bis 10°C mit 0,9 ml Chlorameisensäureethylester in 5 ml abs. Methylenchlorid versetzt. Man rührt bei Raumtemperatur bis die Umsetzung beendet ist (DC-Kontrolle). Dann wäscht man 2 mal mit 50 ml 0,5 N NaOH trocknet und dampft ein. Der Rückstand wird aus MTB-Ether/n-Hexan umkristallisiert.
Ausbeute: 2,50 g (62 % d.Th.), Fp. 211°C.

Analog bzw. gemäß den allgemeinen Angaben zur Herstellung wurden die folgenden Verbindungen der Formel (I-1-c) erhalten:

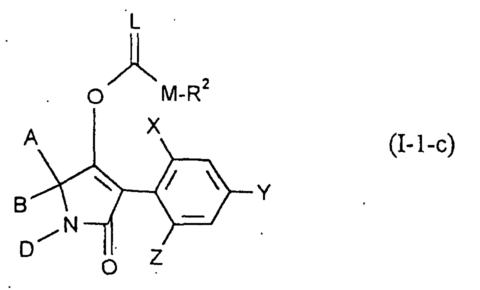

| Bsp.- Nr. | X | Y | Z | A | B | D | L | M | R² | Fp. [°C] | Isomer |
|---|---|---|---|---|---|---|---|---|---|---|---|
| I-1-c-2 | C₂H₅ | CN | C₂H₅ | -(CH₂)₂-CHCH₃-(CH₂)₂- | | H | O | O | Benzyl | 209 | β |
| I-1-c-3 | C₂H₅ | CN | C₂H₅ | -(CH₂)₂-CHCH₃-(CH₂)₂- | | H | O | O | Benzyl | >220 | β |

### Beispiel I-2-a-1

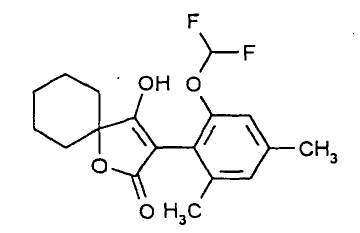

7,7 g (0,02 mol) der Verbindung gemäß Beispiel (III-1) gelöst in wenig THF werden zu 3,4 g (0,03 mol) Kalium-tert.-butylat in 50 ml THF getropft. Anschließend rührt man über Nacht bei Raumtemperatur. Dann gießt man auf Wasser, säuert an und extrahiert mit Methylenchlorid, trocknet die organische Phase und engt ein. Das Rohprodukt wird mit Ether verrieben und abgesaugt. Zur weiteren Reinigung wird an Kieselgel mit dem Laufmittel Cyclohexan/Essigester 1/1 chromatographiert.

### Beispiel I-2-a-2

Analog zu Beispiel I-2-a-1 erhält man die folgende Verbindung: 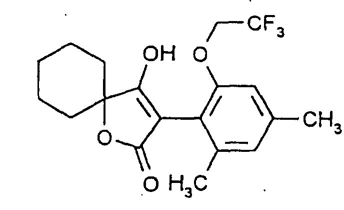 Fp. 185 bis 187°C

### Beispiel I-2-b-1

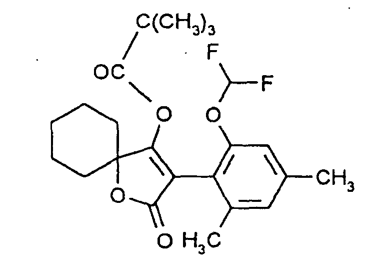

1,2 g (3,5 mmol) der Verbindung gemäß Beispiel I-2-a-1, 0,4 g (3,9 mmol) Triethylamin und eine Spur DABCO werden in 20 ml THF vorgelegt. Dazu tropft man bei 0°C bis 10°C 0,43 g (3,5 mmol) Pivalinsäurechlorid und rührt über Nacht unter Rückfluß. Man engt ein, verteilt zwischen Wasser und Methylenchlorid, trocknet die organische Phase und engt ein. Ausbeute 1,23 g (83 % der Theorie), Fp. 144°C.

### Beispiel I-2-b-2

Analog zu Beispiel I-2-b-1 erhält man die Verbindung: 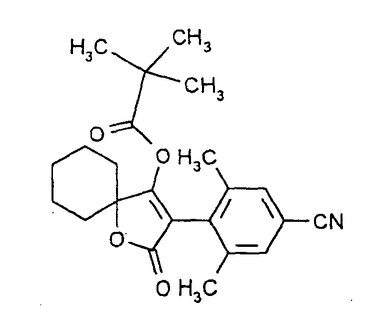 Fp. 143 bis 147°C

### Beispiel I-2-b-3

Analog zu Beispiel I-2-b-1 erhält man die Verbindung:

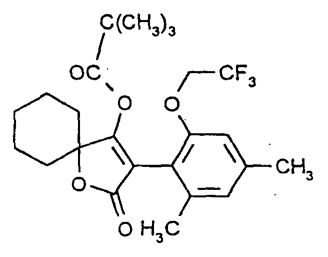

### Beispiel II-1 (β)

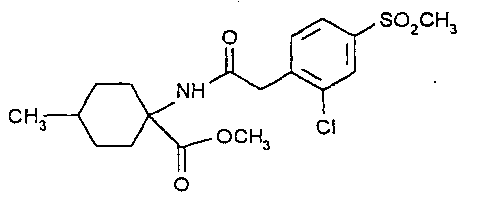

6,77 g (0,0396 Mol) 4-Methylcyclohexylamin-1-carbonsäuremethylester in 65 ml abs. THF werden mit 9,13 ml (0,065 Mol) Triethylamin versetzt. Nach 5 min gibt man 8,1 g (0,0325 Mol) 2-Chlor-4-methylsulfonyl-phenylessigsäure gemäß Beispiel (XXVIII-1) zu, rührt 15 min bei Raumtemperatur, gibt 12,74 ml (0,091 Mol) Triethylamin zu und tropft sofort 3,06 ml Phosphoroxychlorid so zu, daß die Lösung mäßig siedet. Man rührt 30 min unter Rückfluß, gießt auf 300 ml Eiswasser, extrahiert mit Methylenchlorid, trocknet und dampft ein. Der Rückstand wird durch Säulenchromatographie an Kieselgel (Laufmittel Methylenchlorid/Essigester 5/1) gereinigt.
Ausbeute: 5,1 g (39 % d.Th.), Öl.
¹H-NMR (200 MHz, CDCl₃): δ = 0,89 (d, 3H), 3,05 (s, 3H), 3,67 (s, 3H), 3,75 (s, 2H), 5,95 (br, 1H), 7,6 (d, 1H), 7,8 (dd, 1H) 7,97 (d, 1H).

### Beispiel (II-2)

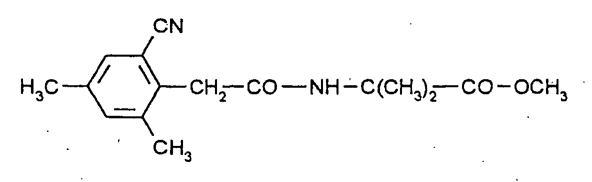

7 g 2-Cyano-4,6-dimethylphenylessigsäure und 8,2 ml Thionylchlorid werden bei 80°C gerührt, bis die Gasentwicklung beendet ist. Das überschüssige Thionylchlorid wird abdestilliert und der Rückstand in 30 ml trockenem THF aufgenommen. Diese Lösung tropft man bei 0°C bis 10°C zum Gemisch von 8,52 g der Verbindung der Formel

H₂N-C(CH₃)₂-CO-OCH₃ x HCl

und 17 ml Triethylamin in 120 ml wasserfreiem THF und rührt eine Stunde bei Raumtemperatur. Anschließend wird eingedampft, der Rückstand in Methylenchlorid aufgenommen, mit 0,5 N HCl gewaschen, getrocknet und erneut eingedampft. Der Rückstand wird aus MTB-Ether/n-Hexan umkristallisiert.
Ausbeute: 7,6 g (71 % der Theorie), Fp. 141°C.

Analog bzw. gemäß den allgemeinen Angaben zur Herstellung wurden die in der folgenden Tabelle aufgeführten Verbindungen der Formel II synthetisiert.

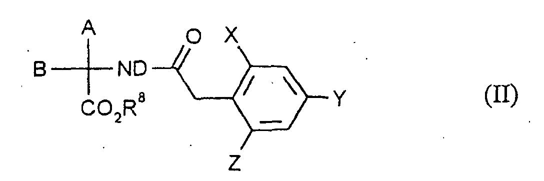

| Bsp.- Nr. | X | Y | Z | A | B | D | R⁸ | Fp.°C | Isomer |
|---|---|---|---|---|---|---|---|---|---|
| II-3 | CH₃ | CN | CH₃ | CH₃ | CH₃ | H | CH₃ | 132 | |
| II-4 | CN | CH₃ | CH₃ | -(CH₂)₂-CHCH₃-(CH₂)₂- | | H | CH₃ | 164 | β |
| II-5 | CH₃ | CN | CH₃ | -(CH₂)₂-CHCH₃-(CH₂)₂- | | H | CH₃ | 195 | β |
| II-6 | C₂H₅ | CN | CH₃ | -(CH₂)₂-CHCH₃-(CH₂)₂- | | H | CH₃ | 161 | β |
| II-7 | OCHF₂ | CH₃ | CH₃ | -(CH₂)₂-CHCH₃-(CH₂)₂- | | H | CH₃ | 121 | β |
| II-8 | OCH₂CF₃ | CH₃ | CH₃ | -(CH₂)₂-CHCH₃-(CH₂)₂- | | H | CH₃ | 142 | β |
| II-9 | C₂H₅ | CN | C₂H₅ | -(CH₂)₂-CHCH₃-(CH₂)₂- | | H | CH₃ | 167 | β |

### Beispiel (III-1)

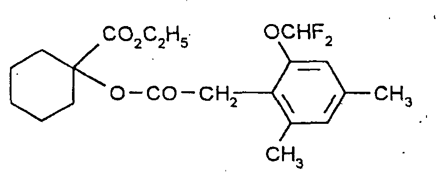

5,33 g (0,031 mol) 1-Hydroxycyclohexancarbonsäureethylester und 7,7 g (0,031 mol) des Carbonsäurechlorids der Formel 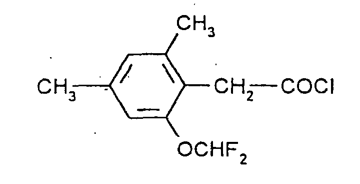 (auf übliche Weise aus der Verbindung gemäß Beispiel XXVIII-c-1 erhalten) werden in 50 ml Toluol über Nacht unter Rückfluß gekocht, anschließend wird eingeengt. Ausbeute 11,9 g (quant.) eines Öls.

### Beispiel III-2

Analog zu Beispiel III-1 erhält man die Verbindung 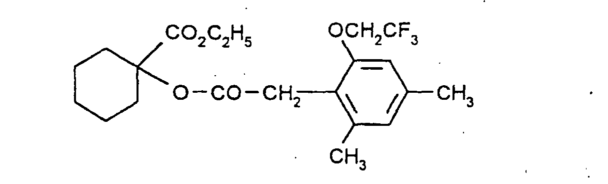 als Öl (Ausbeute 93 % der Theorie).

### Beispiel (XXVIII-1)

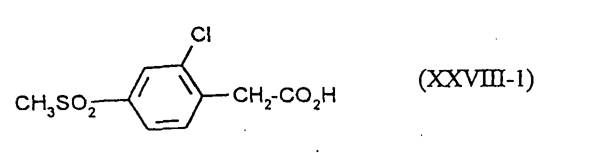

21,3 g (0,1 Mol) 2-Chlor-4-methylsulfonyltoluol (bekannt z.B. aus DE 39 37 282) werden in 43 ml Tetrachlorkohlenstoff gelöst und bei Raumtemperatur 18,3 g (0,1 Mol) N-Bromsuccinimid zugegeben. Danach werden noch 0,1 g AIBN (Azoisobutyronitril) zudosiert und der Ansatz 7 Stunden unter Rückfluß gerührt. Anschließend wird abgesaugt und die Mutterlauge eingeengt. Man erhält 31,3 g 2-Chlor-4-methylsulfonylbenzylbromid mit einer Reinheit von 78,6 % (87 % d.Th.).

31,3 g (87 mMol) 2-Chlor-4-methylsulfonyl-benzylbromid werden mit 1,6 g Tetrabutylammoniumsulfat in 92 ml Methylenchlorid gelöst und bei Raumtemperatur eine Lösung aus 17 g Kaliumcyanid in 70 ml Wasser zugetropft. Man läßt über Nacht bei Raumtemperatur nachrühren, trennt anschließend die Phasen und engt die organische Phase ein. Nach Reinigung über Kieselgel (Laufmittel Methylenchlorid) erhält man 10,5 g 2-Chlor-4-methylsulfonyl-benzylcyanid mit einer Reinheit von 69 % (37 % d.Th.).

In eine Lösung aus 28,1 ml konzentrierter Schwefelsäure und 33,8 ml Wasser werden bei 80-90° 10 g (30 mMol) 2-Chlor-4-methylsulfonyl-benzylcyanid eingetragen und 2 Stunden unter Rückfluß gerührt. Man läßt auf Raumtemperatur abkühlen und gießt den Ansatz auf 75 ml Eiswasser. Der unlösliche Anteil wird abgesaugt, mit Eiswasser gewaschen, getrocknet. Man erhält 3,3 g 2-Chlor-4-methylsulfonyl-phenylessigsäure mit einer Reinheit von 91 % (40,2 % d.Th.).

### Beispiel (XXVIII-c-1)

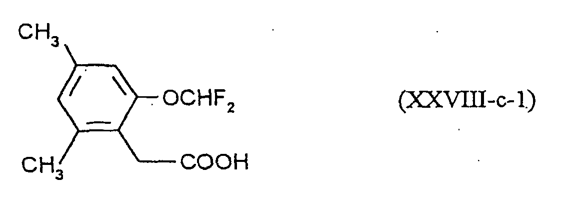

Diese Verbindung wurde auf dem folgenden Weg hergestellt:

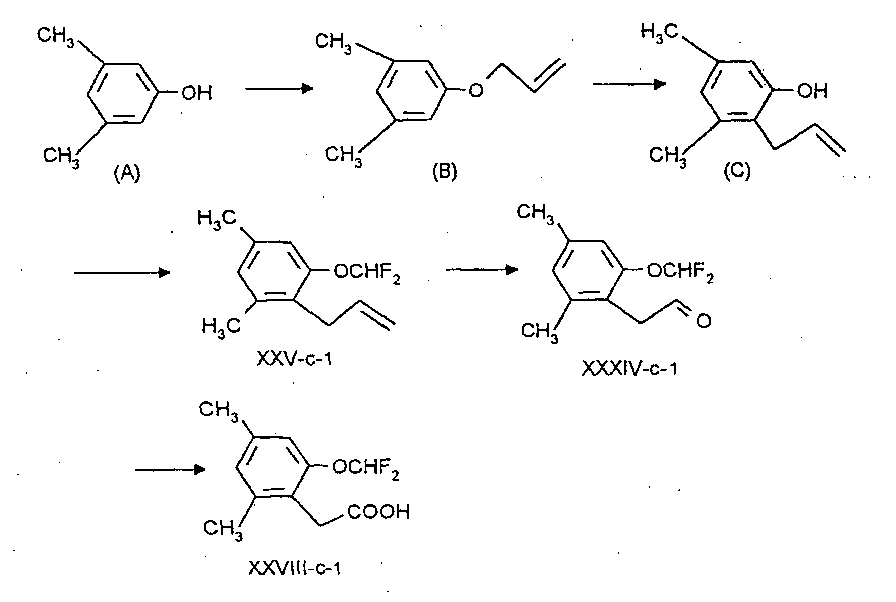

### Allyl-(3,5-dimethylphenyl)ether (B)

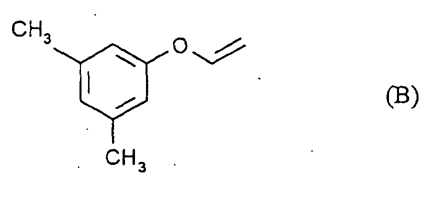

Es wurden 1 000 g 3,5-Dimethylphenol (A) in 1 000 ml Aceton gelöst, 980 g Allylbromid zugetropft und dann 114.8 g Kaliumcarbonat zugesetzt. Die Mischung wurde 18 Stunden zum Rückfluß erhitzt und abgekühlt. Das Kaliumcarbonat wurde filtriert und das Filtrat mit 3 000 ml Wasser versetzt. Es wurde dreimal mit tert.-Butyl-methylether extrahiert, die organischen Phasen vereinigt und zweimal mit je 300 ml 10 %iger Natronlauge gewaschen. Die organischen Phase wurde über Kaliumcarbonat getrocknet, filtriert und eingeengt. Nach Destillation wurden 837 g (64 % der Theorie) Allyl-(3,5-dimethylphenyl)ether mit einem Siedepunkt von 98 bis 110°C bei 10 mbar erhalten.
¹H-NMR (CDCl₃): δ = 6,61 (s, 1H), 6,57 (s, 2H), 6,06 (ddt. 1H), 5,51-5,20 (m, 2H), 4,51 (dm, 2H), 2,30 ppm (s, 3H).

### 3,5 Dimethyl-2-(2-propenyl-1)-phenol (C)

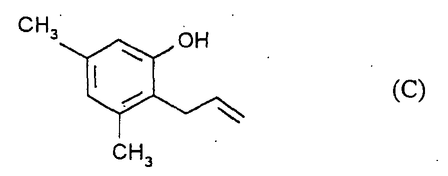

Es wurden 837 g Allyl-(3,5-dimethylphenyl)ether (B) in 1 500 ml Mesitylen gelöst und zwei Tage zum Rückfluß erhitzt. Das Lösungsmittel wurde im Vakuum entfernt und der Rückstand mit tert.-Butylmethylether aufgenommen. Es wurde mehrfach mit Natronlauge extrahiert, die wäßrigen Phasen vereinigt und mit Salzsäure angesäuert. Das sich abscheidende Produkt wurde in tert.-Butylmethylether aufgenommen, über Magnesiumsulfat getrocknet, eingeengt und destilliert. Es wurden insgesamt 619 g (74 % der Theorie) 3,5-Dimethyl-2-(2-propenyl-1)-phenol mit einem Siedepunkt von 145°C bei 30 mbar und einem Schmelzpunkt von 51°C erhalten.
¹H-NMR (CDCl₃): δ = 6,61 (s, 1H), 6,51 (s, 1H), 6,10-5,84 (m, 1H), 5,14-4,95 (m, 2H), 3,39 (d, 2H), 2,25 ppm (s, 7H).

### 3,5-Dimethyl-2-(2-propenyl-1)-phenyldifluormethylether (XXXV-c-1)

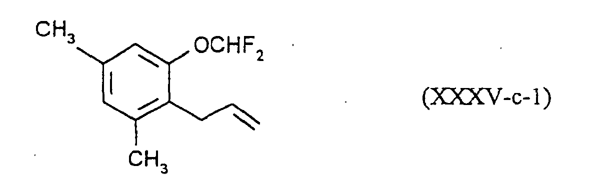

Zu 100 g 3,5 Dimethyl-2-(2-propenyl-1)-phenol (C) in 700 ml Toluol wurden 125 g 45 %ige Natronlauge und 10 g Tetrabutylammoniumbromid zudosiert. Die Mischung wurde auf 90°C erwärmt und anschließend wurden 110 g Chlordifluormethan eingeleitet. Nach dem Abkühlen auf Raumtemperatur wurden 300 ml Wasser zugefügt und die Toluolphase abgetrennt. Die wäßrige Phase wurde zweimal mit 200 ml tert.-Butylmethylether extrahiert und die vereinigten organischen Phasen über Natriumsulfat getrocknet. Es wurde eingeengt und nach der Destillation wurden 58,5 g (45 % der Theorie) 3,5-Dimethyl-2-(2-propenyl-1)-phenyldifluormethylether mit einem Siedepunkt von 55-65°C bei 0,15 mbar erhalten.
¹H-NMR (CDCl₃): δ = 6,83 (s, 1H), 6,75 (s, 1H), 6,37 (t, 1H), 5,91-5,80 (m, 1H), 4,96 (dm, 1H), 4,86 (dm, 1H), 3,48 (d, 2H), 2,26 (s, 3H), 2,24 ppm (s, 3H).

### 2-Difluormethyloxy-4,6-dimethylphenylacetaldehyd (XXXIV-c-1)

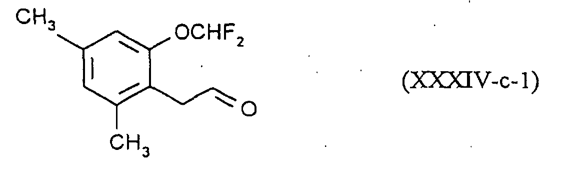

Es wurden 16,5 g 3,5-Dimethyl-2-(2-propenyl-1)-phenyldifluormethylether in 50 ml Dichlormethan gelöst und auf -70°C abgekühlt. Durch die Lösung wurde eine Stunde Ozon geleitet, bis das Olefin nicht mehr zu detektieren war. Anschließend wurde mit einem Stickstoffstrom gespült, 13,7 g Dimethylsulfid zugetropft und 30 min weitergerührt. Die Lösung wurde auf Raumtemperatur erwärmt und noch weitere 30 min nachgerührt. Nachdem die Lösung frei von Peroxiden war, wurde eingeengt und der Rückstand ohne weitere Reinigung in die nächste Reaktion eingesetzt. Es wurden 19,2 g 2-Difluormethyloxy-4,6-dimethylphenylacetaldehyd als Rohprodukt erhalten.
¹H-NMR (CDCl₃): δ = 9,67 (t, 1H), 6,90 (s, 1H), 6,81 (s, 1H), 6,47 (t, 1H), 3,78 (d, 2H) 2,32 (s, 3H), 2,28 ppm (s, 3H)

### 2-Difluormethyloxy-4,6-dimethylphenylessigsäure (XXVIII-c-1)

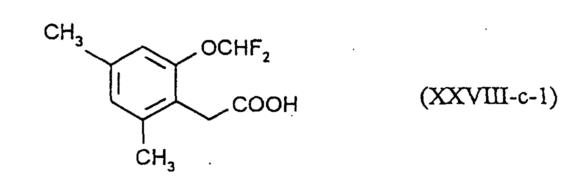

Es wurden 19,2 g 2-Difluormethyloxy-4,6-dimethylphenylacetaldehyd (XXXIV-c-1) als Rohprodukt in 270 ml tert.-Butanol gelöst, mit 90,4 g 2-Methyl-2-buten vermischt und anschließend eine Lösung aus 90 g Natriumdihydrogenphosphat und 42 g Natriumchlorit in 353 ml Wasser bei Raumtemperatur zugetropft. Die Mischung wurde vier Stunden gerührt, dann in 400 ml Essigester eingerührt und die Phasen getrennt. Die wäßrige Phase wurde noch zweimal mit Essigester extrahiert, die vereinigten organischen Phasen über Magnesiumsulfat getrocknet und eingeengt. Nach dem Verrühren mit Hexan wurden 11 g Feststoff isoliert, aus denen 2,5 g (14 % der Theorie über 2 Stufen) 2-Difluormethyloxy-4,6-dimethylphenylessigsäure gewonnen wurden, mit einem Schmelzpunkt von 124-127°C.
¹H-NMR (CDCl₃): δ = 9,50-8,00 (m, 1H), 6,89 (s, 1H), 6,81 (s, 1H), 6,44 (t, 1H), 3,74 (s, 2H), 2,30 (s, 3H), 2,28 ppm (s, 3H).

### Beispiel (XXXV-c-2)

### 3,5-Dimethyl-2-(2-propenyl-1)phenyl-(2,2,2-trifluorethyl)ether (XXXV-c-2)

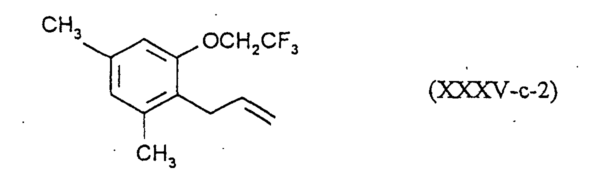

Zu einer Mischung aus 50 g 3,5-Dimethyl-2-(2-propenyl-1)-phenol (C) und 86 g 2,2,2-Trifluorethyltosylat in 450 ml N-Methylpyrrolidon bei 120°C wurden 17 g Natriumhydroxid zudosiert. Es wurde 16 Stunden bei 120°C nachgerührt und anschließend auf 2 l Wasser gegossen. Mit 6 N Salzsäure wurde ein pH-Wert von 2 eingestellt und mit Dichlormethan extrahiert. Die vereinigten organischen Phasen wurden über Natriumsulfat getrocknet, das Lösungsmittel im Vakuum entfernt und der Rückstand destilliert. Es wurden 32 g (43 % der Theorie) 3,5-Dimethyl-2-(2-propenyl-1)phenyl-(2,2,2-trifluorethyl)ether mit einem Siedepunkt von 100°C bei 1 mbar erhalten.

### Beispiel (XXIV-c-2)

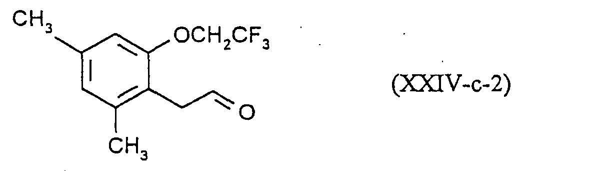

erhalten analog zu Beispiel XXIV-c-1.
Rohausbeute: 28,5 g.

### Beispiel (XXVIII-c-2)

Erhalten analog zu Beispiel (XXVIII-c-1). 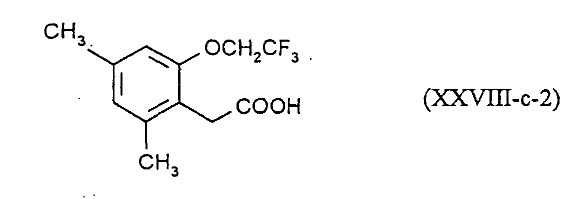 Ausbeute: 12,2 g (52 % der Theorie über 2 Stufen)
Fp. 123-126°C
¹H-NMR (CDCl₃): δ = 6,73 (s, 1H), 6,53 (s, 1H), 4,32 (q, 2H), 3,72 (s, 2H), 2,30 (s, 3H), 2,26 ppm (s, 3H).

### Beispiel (XXX-b-1)

### 4-Cyano-2-ethyl-6-methylphenylessigsäuremethylester

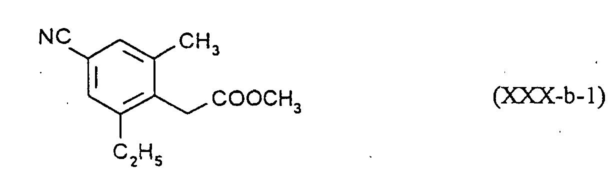

Es wurden 7,5 g 4-Brom-2-ethyl-6-methylphenylessigsäuremethylester in 140 ml Dimethylformamid gelöst, mit 5,5 g Kupfercyanid versetzt und 18 Stunden auf 130°C erhitzt, bis zum vollständigen Umsatz. Danach wurde auf Raumtemperatur gekühlt, mit Wasser verdünnt und das Produkt mehrfach mit tert.-Butyl-methylether extrahiert. Die vereinigten organischen Phasen wurden über Magnesiumsulfat getrocknet, eingeengt und destilliert. Es wurden 3,4 g (52 % der Theorie) 4-Cyano-2-ethyl-6-methylphenylessigsäuremethylester mit einem Siedepunkt von 123-130°C und einem Schmelzpunkt von 65-72°C erhalten.
¹H-NMR (CDCl₃): δ = 7,36 (s, 1H), 7,33 (s, 1H), 3,75 (s, 2H), 3,70 (s, 3H, 2,68 (q, 2H), 2,34 (s, 3H), 1,22 ppm (t, 3H).

### Analog erhielt man

### Beispiel (XXX-b-2)

4-Cyano-2,6-dimethylphenylessigsäuremethylester
Ausbeute: 17,5 g (45 % der Theorie)
Kp. 122-123°C bei 0,09 mbar, Fp. = 50-53°C
¹H-NMR (CDCl₃): δ = 7,32 (s, 2H), 3,73 (s, 2H), 3,70 (s, 3H), 2,34 ppm (s, 6H)

### Beispiel (XXX-b-3)

4-Cyano-2,6-diethylphenylessigsäuremethylester
Kp. 123-133°C / 0,02 mbar; Fp.: 78-81°C
und

### Beispiel (XXX-a-1)

2-Cyano-4,6-dimethylphenylessigsäuremethylester
Ausbeute: 25,3 g (66 % der Theorie)
Kp. 78-81°C
¹H-NMR (CDCl₃): δ = 7,32 (s, 1H), 7,23 (s, 1H), 3,88 (s, 2H), 3,72 (s, 3H), 2,33 (s, 3H), 2,29 ppm (s, 3H).

### Beispiel (XXVIII-a-1)

### 2-Cyano-4,6-dimethylphenylessigsäure

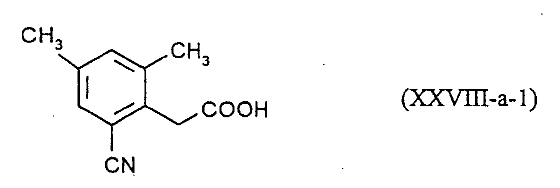

Es wurden 3 g 2-Cyano-4,6-dimethylphenylessigsäuremethylester (XXX-a-1) in 50 ml Tetrahydrofuran gelöst, mit einer Lösung von 355 mg Lithiumhydroxid in 50 ml Wasser versetzt und 18 Stunden bei Raumtemperatur gerührt. Anschließend wurde das Lösungsmittel im Vakuum entfernt, der Rückstand in Wasser aufgenommen und die Neutralstoffe durch Extraktion mit tert.-Butylmethylether entfernt. Die wäßrige Phase wurde auf 0°C abgekühlt und mit 3 molarer Salzsäure sauer gestellt. Das ausgefallene Produkt wurde abgesaugt, mit Wasser gewaschen und getrocknet. Es wurden 2,5 g (86 % der Theorie) 2-Cyano-4,6-dimethylphenylessigsäure mit einem Schmelzpunkt von 155-157°C isoliert.
¹H-NMR (CDCl₃): δ = 7,32 (s, 1H), 7,23 (s, 1H), 3,92 (s, 2H), 2,33 (s, 3H), 2,31 ppm (s, 3H).

### Analog erhielt man

### Beispiel (XXVIII-b-1)

4-Cyano-2-ethyl-6-methylphenylessigsäure
Ausbeute: 1,8 g (64 % der Theorie)
Fp. = 157-161°C
IR: 2222 cm⁻¹ (CN)
¹H-NMR (CDCl₃): δ = 7,38 (s, 1H), 7,34 (s, 1H), 3,77 (s, 2H), 2,68 (q, 2H), 2,35 (s, 3H), 1,22 ppm (t, 3H)

### Beispiel (XXVIII-b-2)

4-Cyano-2,6-dimethylphenylessigsäure (XXVIII-b-2)
Ausbeute: 11,8 g (91 % der Theorie)
Fp. = 202-206°C
¹H-NMR (CDCl₃): δ = 7,34 (s, 2H), 3,77 (s, 2H), 2,36 ppm (s, 6H)
und

### Beispiel (XXVIII-b-3)

4-Cyano-2,6-diethylphenylessigsäure
Fp.: 158-162°C.

### Anwendungsbeispiele

### Beispiel A

| Plutella-Test | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet; daß keine Raupen abgetötet wurden.

In diesem Test bewirkten z.B. die Verbindungen gemäß den Herstellungsbeispielen I-1-a-1 und I-1-c-1 bei einer beispielhaften Wirkstoffkonzentration von 0,1 % eine Abtötung von mindestens 85 % nach 7 Tagen.

### Beispiel B

### Nephotettix-Test

| | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit der Grünen Reiszikase (Nephotettix cinticeps) besetzt, solange die Blätter noch feucht sind.

Nach der gewünschten Zeit wird die Abtötung in % bestimmt. Dabei bedeutet 100 %, daß alle Zikaden abgetötet wurden; 0 % bedeutet; daß keine Zikaden abgetötet wurden.

In diesem Test bewirkte z.B. die Verbindung gemäß den Herstellungsbeispielen 1-1-a-1, I-1-a-4, I-1-a-5 und I-1-a-6 bei einer beispielhaften Wirkstoffkonzentration von 0,1 % eine Abtötung von 100 % nach 6 Tagen.

### Beispiel C

### Pre-emergence-Test

| | | |
|---|---|---|
| Lösungsmittel | 3 Gewichtsteile | Aceton |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

In diesem Test bewirkten z.B. die Verbindungen gemäß den Herstellungsbeispielen I-1-a-1 und I-1-c-1 bei einer beispielhaften Aufwandmenge von 4000 g/ha bei guter Verträglichkeit durch Beta vulgaris eine Schädigung von 80 % gegenüber Alopecurus myosuroides.

Die Verbindung gemäß Herstellungsbeispiel I-1-a-6 bewirkte bei einer beispielhaften Aufwandmenge von 250 g/ha bei guter Verträglichkeit durch Mais eine Schädigung von 80 % gegenüber Abutilon.

Die Verbindung gemäß Herstellungsbeispiel I-1-a-4 bewirkte bei einer beispielhaften Aufwandmenge von 250 g/ha bei guter Verträglichkeit durch Soja eine Schädigung von jeweils 100 % gegenüber Alopecusus, Digitatia, Echinocloa, Chenopodium und Veronica.

### Beispiel D

### Myzus-Test

| | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der grünen Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden, 0 % bedeutet, daß keine Blattläuse abgetötet wurden.

In diesem Test bewirkten z.B. die Verbindungen gemäß den Herstellungsbeispielen I-1-a-4, I-1-a-5 und I-1-a-6 bei einer beispielhaften Wirkstoffkonzentration von 0,01 % eine Abtötung von mindestens 95 % nach 6 Tagen.

### Beispiel E

### Tetranychus-Test (OP-resistent)

| | | |
|---|---|---|
| Lösungsmittel | 7 Gewichtsteile | Dimethylformamid |
| Emulgator | 1 Gewichtsteil | . Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnentriebe (Phaseolus vulgaris), die stark von der gemeinen Spinnmilbe (Tetranychus urticae) befallen sind, werden in eine Wirkstoffzubereitung der gewünschten Konzentration getaucht.

Nach der gewünschten Zeit wird die Abtötung in % bestimmt. Dabei bedeutet 100 %, daß alle Spinnmilben abgetötet wurden, 0 % bedeutet, daß keine Spinnmilben abgetötet wurden.

In diesem Test zeigten z.B. die Verbindungen gemäß den Herstellungsbeispielen I-1-a-4, I-1-a-5 und I-1-a-6 bei einer beispielhaften Wirkstoffkonzentration von 0,1 % eine Wirkung von 100 % nach 13 Tagen.

### Beispiel F

### Test mit Boophilus microplus resistent/SP-resistenter Parkhurst-Stamm

| | |
|---|---|
| Testtiere | adulte gesogene Weibchen |
| Lösungsmittel | Dimethylsulfoxid |

20 mg Wirkstoff werden in 1 ml Dimethylsulfoxid gelöst, geringere Konzentrationen werden durch verdünnen in dem gleichen Lösungsmittel hergestellt.

Der Test wird in 5-fach-Bestimmung durchgeführt. 1 µl der Lösungen wird in das Abdomen injiziert, die Tiere in Schalen überführt und in einem klimatisierten Raum aufbewahrt. Die Wirkung wird über die Hemmung der Eiablage bestimmt. Dabei bedeutet 100 %, daß keine Zecke gelegt hat.

In diesem Test zeigte z.B. die Verbindung gemäß den Herstellungsbeispiel I-1-a-1 bei einer beispielhaften Wirkstoffkonzentration von 20 µg/Tier eine Wirkung von 100 %.

### Beispiel G

### Post-emergence-Test

| | | |
|---|---|---|
| Lösungsmittel | 5 Gewichtsteile | Aceton |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5-15 cm haben, so daß die jeweils gewünschten Wirkstoffmengen pro Flächeneinheit ausgebracht werden. Die Konzentration der Spritzbrühe wird so gewählt, daß in 2 000 1 Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test bewirkte z.B. die Verbindung gemäß den Herstellungsbeispiel I-1-a-5 bei einer beispielhaften Aufwandmenge von 125 g/ha bei guter Verträglichkeit durch Soja eine Schädigung von mindestens 80 % gegenüber Alopecurus, Avenafatua und Setaria.

## Patentansprüche

1. Verbindungen der Formel (I) 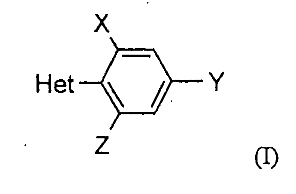 in welcher
X für Halogen, C₁-C₆-Alkyl, C₁-C₆-Halogenalkyl, C₁-C₆-Halogenalkoxy oder Cyano steht,
Y für C₁-C₆-Alkyl, C₁-C₆-Alkylsulfonyl, C₁-C₆-Halogenalkyl, C₁-C₆-Halogenalkoxy oder Cyano steht,
Z für C₁-C₆-Alkyl steht, wobei mindestens einer der Substituenten X und Y nicht für Alkyl oder Halogenalkyl steht,
Het für eine der Gruppen 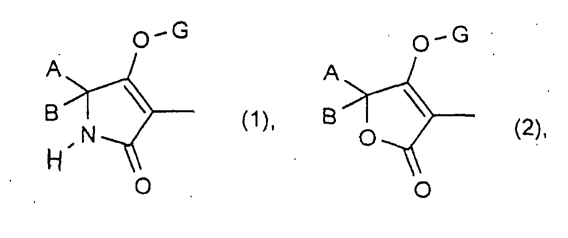 steht,
A, B und das Kohlenstoffatom, an das sie gebunden sind, für gesättigtes C₃-C₁₀-Cycloalkyl stehen, in welchem gegebenenfalls ein Ringglied gegebenenfalls einfach oder mehrfach durch C₁-C₈-Alkyl oder C₁-C₈-Alkoxy, substituiert ist,
G für Wasserstoff (a) oder für eine der Gruppen 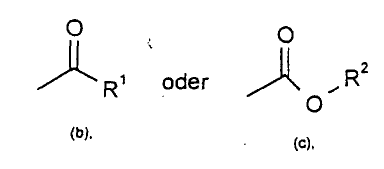 steht,
in welchen
R¹ für C₁-C₂₀-Alkyl oder C₁-C₈-Alkoxy-C₁-C₈-alkyl steht,
R² für C₁-C₂₀-Alkyl steht.

2. Verbindungen der Formel (I) gemäß Anspruch 1, in welcher
X für Fluor, Chlor, Brom, C₁-C₄-Alkyl, C₁-C₄-Halogenalkyl, C₁-C₄-Halogenalkoxy oder Cyano steht,
Y für C₁-C₄-Alkyl, C₁-C₄-Alkylsulfonyl, C₁-C₄-Halogenalkyl, C₁-C₄-Halogenalkoxy oder Cyano steht,
Z für C₁-C₄-Alkyl steht, wobei mindestens einer der Substituenten X und Y nicht für Alkyl oder Halogenalkyl steht,
Het für eine der Gruppen 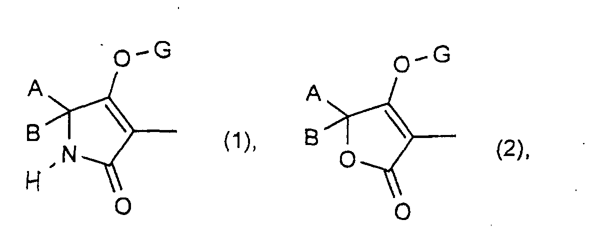 steht
A, B und das Kohlenstoffatom an das sie gebunden sind, für gesättigtes C₃-C₈-Cycloalkyl steht, in welchem gegebenenfalls ein Ringglied gegebenenfalls durch C₁-C₆-Alkyl, oder C₁-C₆-Alkoxy substituiert ist,
G für Wasserstoff (a) oder für eine der Gruppen 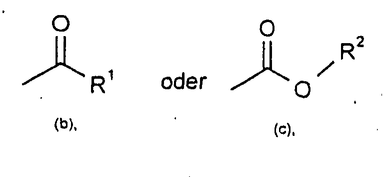 steht,
in welchen
R¹ für C₁-C₁₆-Alkyl oder C₁-C₆-Alkoxy-C₁-C₆-alkyl steht,
R² für C₁-C₁₆-Alkyl steht,

3. Verbindungen der Formel (I) gemäß Anspruch 1, in welcher
X für Fluor, Chlor, Brom, Methyl, Ethyl, Propyl, iso-Propyl, Trifluormethyl, Difluormethoxy, Trifluormethoxy, oder Cyano steht,
Y für Methyl, Ethyl, n-Propyl, i-Propyl, n-Butyl, i-Butyl, tert.-Butyl, Trifluormethyl, Methylsulfonyl, Difluormethoxy, Trifluormethoxy oder Cyano steht,
Z für Methyl, Ethyl, n-Propyl, i-Propyl, n-Butyl, i-Butyl oder tert.-Butyl steht, wobei mindestens einer der Substituenten X und Y nicht für Alkyl oder Halogenalkyl steht,
Het für eine der Gruppen 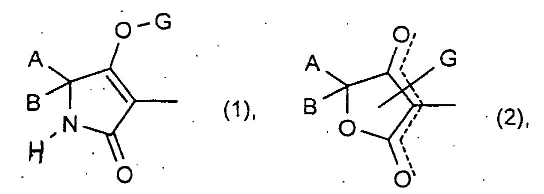 steht,
A, B und das Kohlenstoffatom an das sie gebunden sind, für gesättigtes C₃-C₈-Cycloalkyl stehen, in welchem gegebenenfalls ein Ringglied gegebenenfalls durch Methyl, Ethyl, Propyl, Isopropyl, Butyl, iso-Butyl, sec.-Butyl, tert.-Butyl, Methoxy, Ethoxy, Propoxy, iso-Propoxy, Butoxy, iso-Butoxy, sek.-Butoxy oder tert.-Butoxy, substituiert ist,
G für Wasserstoff (a) oder für eine der Gruppen 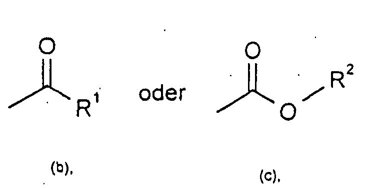 steht, in welchen
R¹ für C₁-C₁₄-Alkyl oder C₁-C₄-Alkoxy-C₁-C₆-alkyl steht,
R² für C₁-C₁₄-Alkyl steht.

4. Verfahren zur Herstellung von Verbindungen der Formel (I) gemäß Anspruch 1, **dadurch gekennzeichnet, dass** man zum Erhalt von
(A) Verbindungen der Formel (I-1-a) 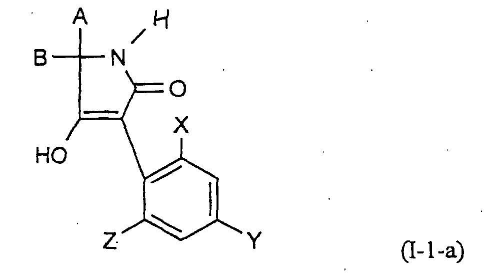 in welcher
A, B, X, Y und Z die oben angegebenen Bedeutungen haben,
N-Acylaminosäureester der Formel (II) 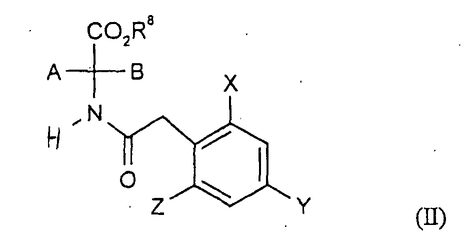 in welcher
A, B, X, Y und Z die oben angegebenen Bedeutungen haben,
und
R⁸ für Alkyl steht,
in Gegenwart eines Verdünnungsmittels und in Gegenwart einer Base intramolekular kondensiert,
(B) Verbindungen der Formel (I-2-a) 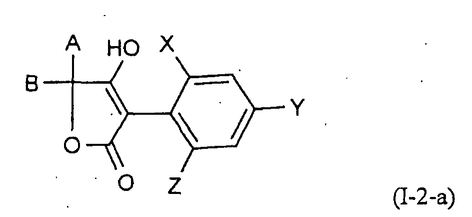
in welcher
A, B, X, Y und Z die oben angegebenen Bedeutungen haben,
Carbonsäureester der Formel (III) 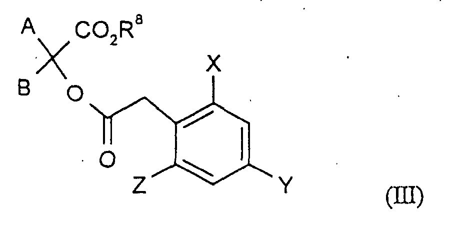 in welcher
A, B, X, Y, Z und R⁸ die oben angegebenen Bedeutungen haben,
in Gegenwart eines Verdünnungsmittels und in Gegenwart einer Base intramolekular kondensiert,
und gegebenenfalls die so erhaltenen Verbindungen der Formeln (I-1-a) oder (I-2-a),
Gα) mit Säurechloriden der Formel (X) 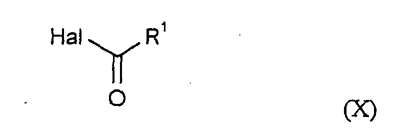 in welcher
R¹ die oben angegebene Bedeutung hat und
Hal für Halogen steht
oder
β) mit Carbonsäureanhydriden der Formel (XI)
R¹-CO-O-CO-R¹ (XI)
in welcher
R¹ die oben angegebene Bedeutung hat,
gegebenenfalls in Gegenwart eines Verdünnungsmittels und gegebenenfalls in Gegenwart eines Säurebindemittels umsetzt oder
(H) mit Chlorameisensäureestern oder Chlorameisensäurethiolestern der Formel (XII)
R²-O-CO-Cl (XII)
in welcher
R² die oben angegebenen Bedeutungen hat,
gegebenenfalls in Gegenwart eines Verdünnungsmittels und gegebenenfalls in Gegenwart eines Säurebindemittels umsetzt.

5. Verbindungen der Formel (II) 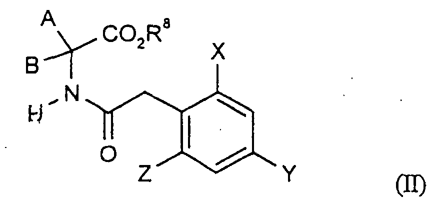 in welcher
A, B, X, Y und Z die in Anspruch 1 angegebenen Bedeutungen haben und
R⁸ für Alkyl steht.

6. Verbindungen der Formel (XXIII) 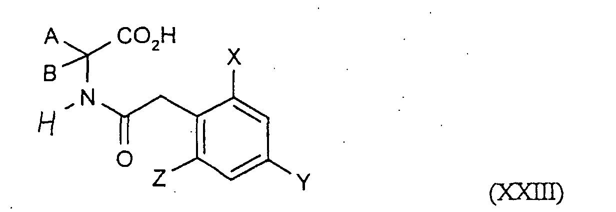 in welcher
A, B, X, Y und Z die in Anspruch 1 angegebenen Bedeutungen haben.

7. Verbindungen der Formel (XXVI) 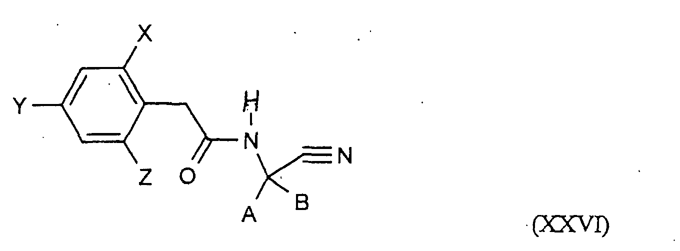 in welcher
A, B, X, Y und Z die in Anspruch 1 angegebenen Bedeutungen haben.

8. Verbindungen der Formel (III) 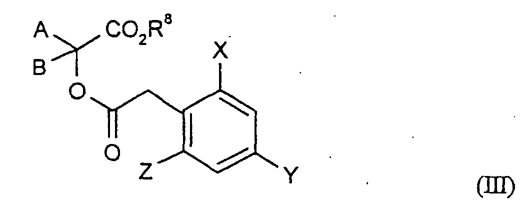 in welcher
A, B, X, Y und Z die in Anspruch 1 angegebenen Bedeutungen haben und
R⁸ für Alkyl steht.

9. Schädlingsbekämpfungsmittel und Unkrautbekämpfungsmittel, **gekennzeichnet durch** einen Gehalt an mindestens einer Verbindung der Formel (I) gemäß Anspruch 1.

10. Verwendung von Verbindungen der Formel (I) gemäß Anspruch 1 zur Bekämpfung von Schädlingen und Unkräutern, ausgenommen die Verwendung in Verfahren zur Behandlung des menschlichen und tierischen Körpers.

11. Verfahren zur Bekämpfung von Schädlingen und Unkräutern, **dadurch gekennzeichnet, dass** man Verbindungen der Formel (I) gemäß Anspruch 1 auf Schädlinge und/oder ihren Lebensraum einwirken lässt oder auf Unkräuter und/oder ihren Lebensraum einwirken lässt, ausgenommen Verfahren zur Behandlung des menschlichen und tierischen Körpers.

12. Verfahren zur Herstellung von Schädlingsbekämpfungsmitteln und Unkrautbekämpfungsmitteln, **dadurch gekennzeichnet, dass** man Verbindungen der Formel (I) gemäß Anspruch 1 mit Streckmitteln und/oder oberflächenaktiven Mitteln vermischt.

13. Verwendung von Verbindungen der Formel (I) gemäß Anspruch 1 zur Herstellung von Schädlingsbekämpfungsmitteln und Unkrautbekämpfungsmitteln.

14. Verbindungen der Formel (XXII-a) 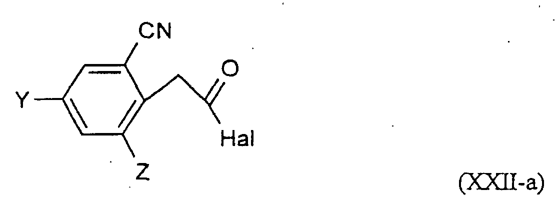 in welcher
Hal für Chlor oder Brom steht und
Y und Z die in Anspruch 1 angegebenen Bedeutungen haben, aber nicht gleichzeitig für Wasserstoff stehen.

## Claims

1. Compounds of the formula (I) 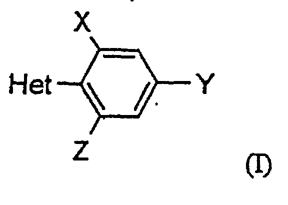 in which
X represents halogen, C₁-C₆-alkyl, C₁-C₆-halogenoalkyl, C₁-C₆-halogenoalkoxy, or cyano,
Y represents C₁-C₆-alkyl, C₁-C₆-alkylsulphonyl, C₁-C₆-halogenoalkyl, C₁-C₆-halogenoalkoxy, or cyano,
Z represents C₁-C₆-alkyl, where at least one of the substituents X and Y does not represent alkyl or halogenoalkyl,
Het represents one of the groups 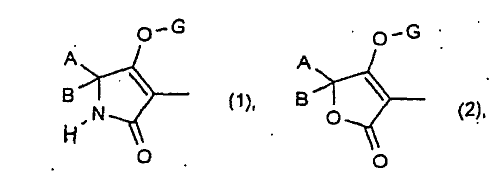
A, B and the carbon atom to which they are bonded represent saturated C₃-C₁₀-cycloalkyl in which optionally one ring member is optionally monosubstituted or polysubstituted by C₁-C₈-alkyl or C₁-C₈-alkoxy,
G represents hydrogen (a) or one of the groups 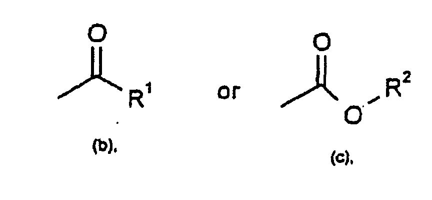 in which
R¹ represents C₁-C₂₀-alkyl or C₁-C₈-alkoxy-C₁-C₈-alkyl,
R² represents C₁-C₂₀-alkyl.

2. Compounds of the formula (I) according to Claim 1 in which
X represents fluorine, chlorine, bromine, C₁-C₄-alkyl, C₁-C₄-halogenoalkyl, C₁-C₄-halogenoalkoxy, or cyano,
Y represents C₁-C₄-alkyl, C₁-C₄-alkylsulphonyl, C₁-C₄-halogenoalkyl, C₁-C₄-halogenoalkoxy, or cyano,
Z represents C₁-C₄-alkyl, where at least one of the substituents X and Y does not represent alkyl or halogenoalkyl,
Het represents one of the groups 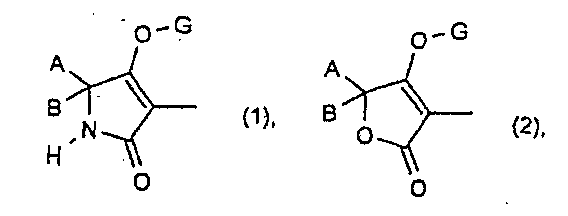
A, B and the carbon atom to which they are bonded represent saturated C₃-C₈-cycloalkyl in which optionally one ring member is optionally substituted by C₁-C₆-alkyl or C₁-C₆-alkoxy,
G represents hydrogen (a) or one of the groups 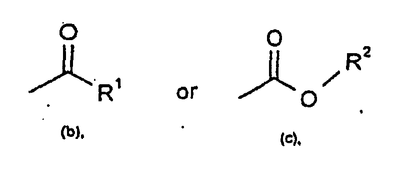 in which
R¹ represents C₁-C₁₆-alkyl or C₁-C₆-alkoxy-C₁-C₆-alkyl,
R² represents C₁-C₁₆-alkyl.

3. Compounds of the formula (I) according to Claim 1 in which
X represents fluorine, chlorine, bromine, methyl, ethyl, propyl, iso-propyl, trifluoromethyl, difluoromethoxy, trifluoromethoxy or cyano,
Y represents methyl, ethyl, n-propyl, i-propyl, n-butyl, i-butyl, tert-butyl, trifluoromethyl, methylsulphonyl, difluoromethoxy, trifluoromethoxy or cyano,
Z represents methyl, ethyl, n-propyl, i-propyl, n-butyl, i-butyl or tert-butyl, where at least one of the substituents X and Y does not represent alkyl or halogenoalkyl,
Het represents one of the groups 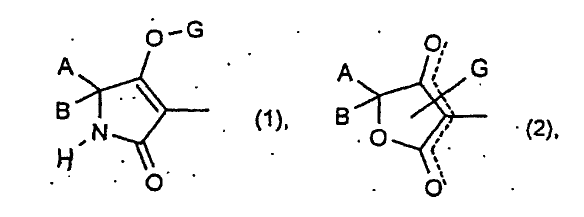
A, B and the carbon atom to which they are bonded represent saturated C₃-C₈-cycloalkyl in which optionally one ring member is optionally substituted by methyl, ethyl, propyl, isopropyl, butyl, iso-butyl, sec-butyl, tert-butyl, methoxy, ethoxy, propoxy, iso-propoxy, butoxy, iso-butoxy, sec-butoxy or tert-butoxy,
G represents hydrogen (a) or one of the groups 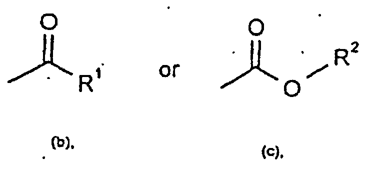 in which
R¹ represents C₁-C₁₄-alkyl or C₁-C₄-alkoxy-C₁-C₆-alkyl,
R² represents C₁-C₁₄-alkyl.

4. Process for the preparation of compounds of the formula (I) according to Claim 1, **characterized in that**, to obtain
(A) compounds of the formula (I-1-a) 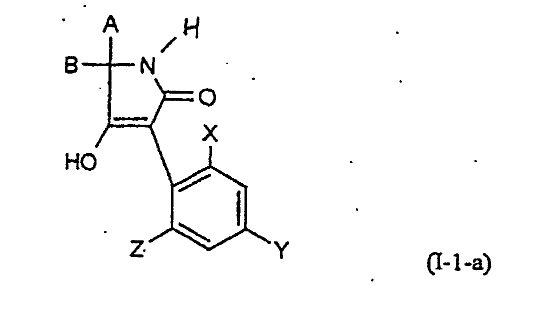 in which
A, B, X, Y and Z have the abovementioned meanings,
N-acylamino acid esters of the formula (II) 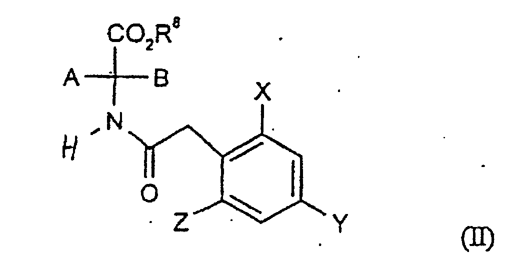 in which
A, B, X, Y and Z have the abovementioned meanings
and
R⁸ represents alkyl
are subjected to an intramolecular condensation reaction in the presence of a diluent and in the presence of a base,
(B) compounds of the formula (I-2-a) 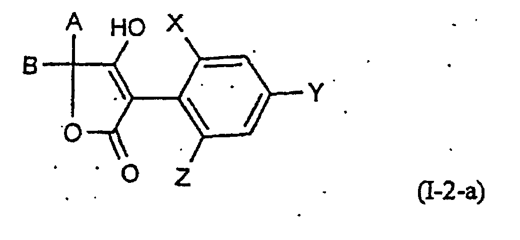 in which
A, B, X, Y and Z have the abovementioned meanings,
carboxylic esters of the formula (III) 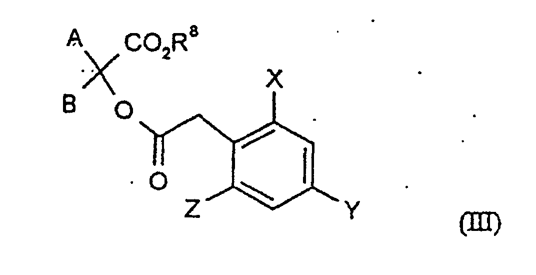 in which
A, B, X, Y, Z and R⁸ have the abovementioned meanings
are subjected to an intramolecular condensation reaction in the presence of a diluent and in the presence of a base,
and, if appropriate, the resulting compounds of the formulae (I-1-a) or (I-2-a),
Gα) are reacted with acid chlorides of the formula (X) 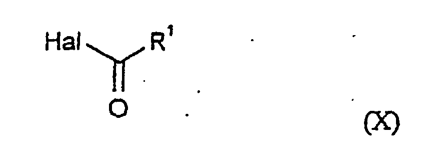 in which
R¹ has the abovementioned meaning and
Hal represents halogen
or
β) are reacted with carboxylic anhydrides of the formula (XI)
R¹-CO-O-CO-R¹ (XI)
in which
R¹ has the abovementioned meaning,
if appropriate in the presence of a diluent and if appropriate in the presence of an acid-binding agent, or
(H) are reacted with chloroformic esters or chloroformic thiolesters of the formula (XII)
R²-O-CO-Cl (XII)
in which
R² has the abovementioned meanings,
if appropriate in the presence of a diluent and if appropriate in the presence of an acid-binding agent.

5. Compounds of the formula (II) 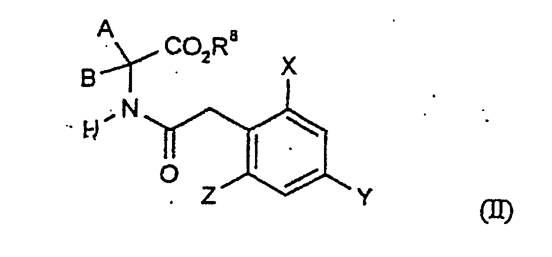 in which
A, B, X, Y and Z have the meanings given in Claim 1 and
R⁸ represents alkyl.

6. Compounds of the formula (XXIII) 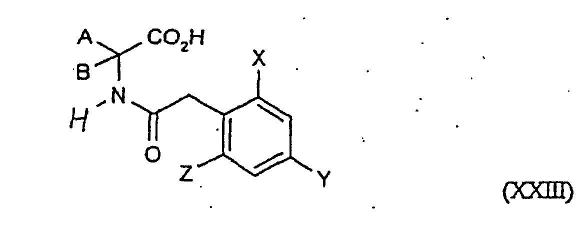 in which
A, B, X, Y and Z have the meanings given in Claim 1.

7. Compounds of the formula (XXVI) 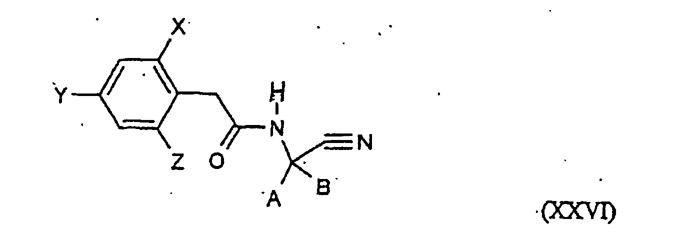 in which
A, B, X, Y and Z have the meanings given in Claim 1.

8. Compounds of the formula (III) 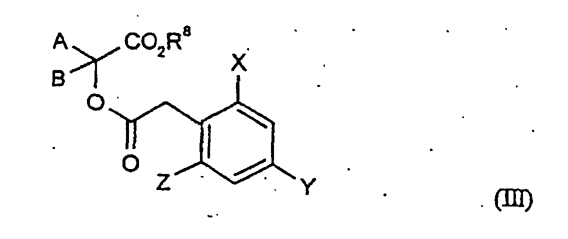 in which
A, B, X, Y and Z have the meanings given in Claim 1 and
R⁸ represents alkyl.

9. Pesticides and herbicides, **characterized in that** they comprise at least one compound of the formula (I) according to Claim 1.

10. Use of compounds of the formula (I) according to Claim 1 for combating pests and weeds, except the use in methods for treating the human and animal body.

11. Method of combating pests and weeds, **characterized in that** compounds of the formula (I) according to Claim 1 are allowed to act on pests and/or their environment or on weeds and/or their environment, except methods for treating the human and animal body.

12. Process for the preparation of pesticides and herbicides, **characterized in that** compounds of the formula (I) according to Claim 1 are mixed with extenders and/or surface-active agents.

13. Use of compounds of the formula (I) according to Claim 1 for the preparation of pesticides and herbicides.

14. Compounds of the formula (XXII-a) 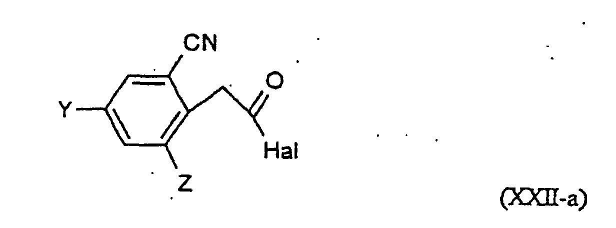 in which
Hal represents chlorine or bromine and
Y and Z have the meanings given in Claim 1, but do not simultaneously represent hydrogen.

## Revendications

1. Composés de formule (I) 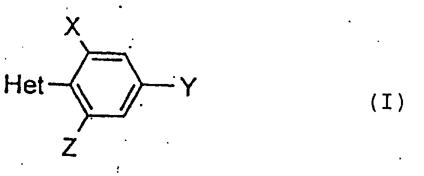 dans laquelle
X désigne un halogène, un reste alkyle en C₁ à C₆, halogénalkyle en C₁ à C₆, halogénalkoxy en C₁ à C₆ ou cyano,
Y est un reste alkyle en C₁ à C₆, alkylsulfonyle en C₁ à C₆, halogénalkyle en C₁ à C₆, halogénalkoxy en C₁ à C₆ ou cyano,
Z est un reste alkyle en C₁ à C₆, l'un ou un des substituants X et Y ne représentant pas un reste alkyle ou halogénalkyle,
Het représente l'un des groupes 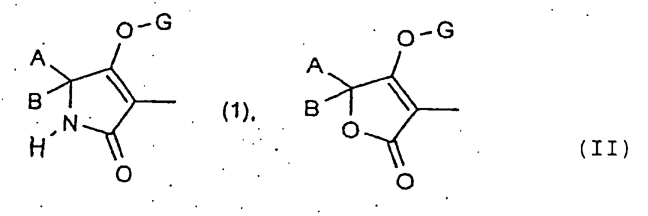
A, B et l'atome de carbone auquel ils sont liés forment un groupe cyclo-alkyle saturé en C₃ à C₁₀ dans lequel, le cas échéant, un chaînon est éventuellement substitué une ou plusieurs fois par un radical alkyle en C₁ à C₈ ou alkoxy en C₁ à C₈,
G représente l'hydrogène (a) ou l'un des groupes 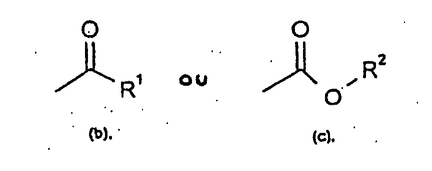 dans lesquels
R¹ est un reste alkyle en C₁ à C₂₀ ou (alkoxy en C₁ à C₈)-(alkyle en C₁ à C₈),
R² est un reste alkyle en C₁ à C₂₀.

2. Composés de forme (I) suivant la revendication 1, formule dans laquelle
X représente le fluor, le chlore, le brome, un reste alkyle en C₁ à C₄, halogénalkyle en C₁ à C₄, halogénalkoxy en C₁ à C₄ ou cyano,
Y est un reste alkyle en C₁ à C₄, alkylsulfonyle en C₁ à C₄, halogénalkyle en C₁ à C₄, halogénalkoxy en C₁ à C₄ ou cyano,
Z est un reste alkyle en C₁ à C₄, l'un au moins des substituants X et Y ne représentant pas un reste alkyle ou halogénalkyle,
Het représente l'un des groupes 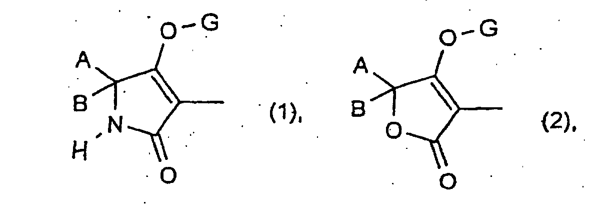
A, B et l'atome de carboné auquel ils sont liés forment un reste cyclo-alkyle saturé en C₃ à C₈ dont au moins un chaînon est éventuellement substitué par un radical alkyle en C₁ à C₆ ou alkoxy en C₁ à C₆,
G représente l'hydrogène (a) ou l'un des groupes 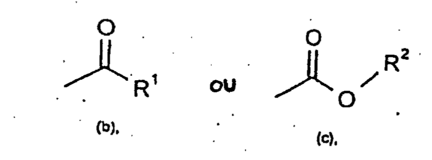 dans lesquels
R¹ est un reste alkyle en C₁ à C₁₆ ou (alkoxy en C₁ à C₆)-(alkyle en C₁ à C₆),
R² est un reste alkyle en C₁ à C₁₆.

3. Composés de formule (I) suivant la revendication 1, formule dans laquelle
X représente le fluor, le chlore, le brome, un reste méthyle, éthyle, propyle, isopropyle, trifluorométhyle, difluorométhoxy, trifluorométhoxy ou cyano,
Y est un reste méthyle, éthyle, n-propyle, isopropyle, n-butyle, isobutyle, tertiobutyle, trifluorométhyle, méthylsulfonyle, difluorométhoxy, trifluorométhoxy ou cyano,
Z est un reste méthyle, éthyle, n-propyle, isopropyle, n-butyle, isobutyle ou tertiobutyle, l'un au moins des substituants X et Y n'étant pas un reste alkyle ou halogénalkyle,
Het représente l'un des groupes 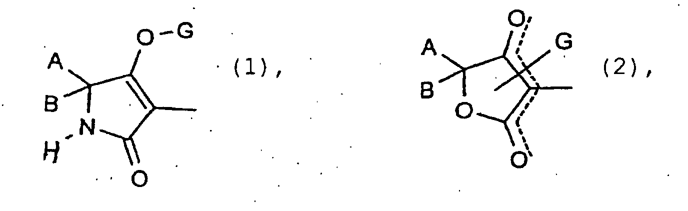
A, B et l'atome de carbone auquel ils sont liés forment un reste cyclo-alkyle saturé en C₃ à C₈ dans lequel, le cas échéant, un chaînon est éventuellement substitué par un radical méthyle, éthyle, propyle, isopropyle, butyle, isobutyle, sec.-butyle, tertiobutyle, méthoxy, éthoxy, propoxy, isopropoxy, butoxy, isobutoxy, sec.-butoxy ou tertiobutoxy,
G représente l'hydrogène (a) ou l'un des groupes 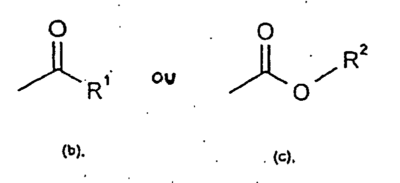 dans lesquels
R¹ est un reste alkyle en C₁ à C₁₄ ou (alkoxy en C₁ à C₄)-(alkyle en C₁ à C₆),
R² est un reste alkyle en C₁ à C₁₄.

4. Procédé de production de composés de formule (I) suivant la revendication 1, **caractérisé en ce que** pour obtenir :
(A) des composés de formule (I-1-a) 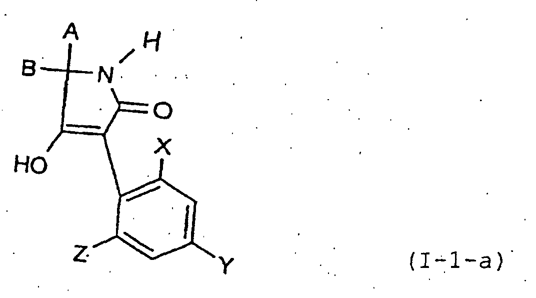 dans laquelle
A, B, X, Y et Z ont les définitions indiquées ci-dessus,
on effectue la condensation intramoléculaire d'esters de N-acylaminoacides de formule (II) 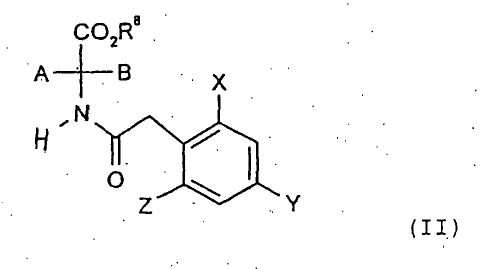 dans laquelle
A, B, X, Y et Z ont les définitions indiquées ci-dessus, et
R⁸ est un reste alkyle,
en présence d'un diluant et en présence d'une base,
(B) des composés de formule (I-2-a) 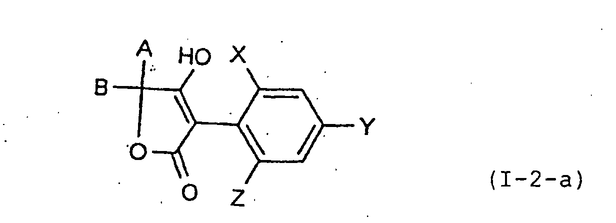 dans laquelle
A, B, X, Y et Z ont les définitions indiquées ci-dessus, on effectue la condensation intramoléculaire d'esters d'acides carboxyliques de formule (III) 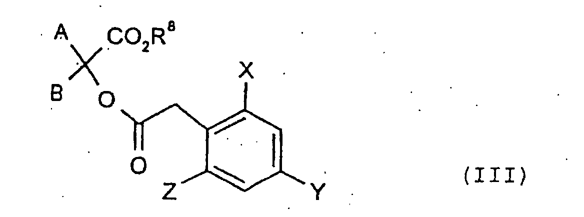 dans laquelle
A, B, X, Y, Z et R⁸ ont les définitions indiquées ci-dessus,
en présence d'un diluant et en présence d'une base,
et le cas échéant, on fait réagir les composés ainsi obtenus de formules (I-1-a) ou (I-2-a),
Gα) avec des chlorures d'acide de formule (X) 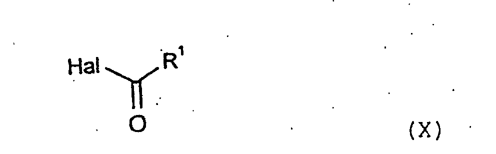 dans laquelle
R¹ a la définition indiquée ci-dessus et
Hal est un halogène
ou bien
β) avec des anhydrides d'acides carboxyliques de formule (XI)
R¹-CO-O-CO-R¹ (XI)
dans laquelle
R¹ a la définition indiquée ci-dessus,
éventuellement en présence d'un diluant et, le cas échéant, en présence d'un accepteur d'acide, ou bien
(H) avec des esters d'acide chloroformique ou des thioesters d'acide chloroformique de formule (XII)
R²-O-OC-Cl (XII)
dans laquelle
R² a la définition indiquée ci-dessus,
éventuellement en présence d'un diluant et, le cas échéant, en présence d'un accepteur d'acide.

5. Composés de formule (II) 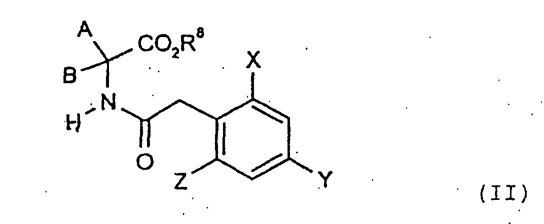 dans laquelle
A, B, X, Y et Z ont les définitions indiquées dans la revendication 1 et
R⁸ est un reste alkyle.

6. Composés de formule (XXIII) 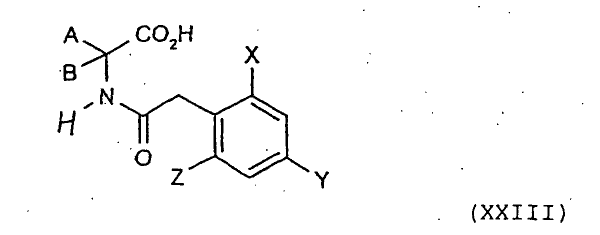 dans laquelle
A, B, X, Y et Z ont les définitions indiquées dans la revendication 1.

7. Composés de formule (XXVI) 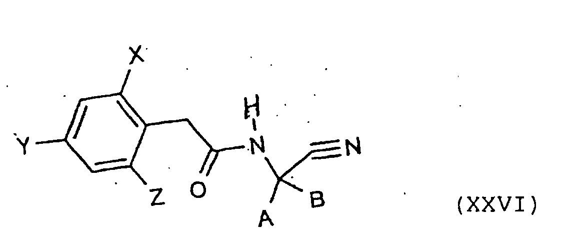 dans laquelle
A, B, X, Y et Z ont les définitions indiquées dans la revendication 1.

8. Composés de formule (III) 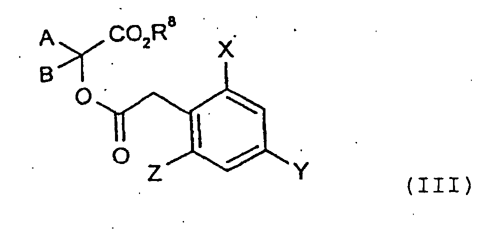 dans laquelle
A, B, X, Y et Z ont les définitions indiquées dans la revendication 1 et
R⁸ est un reste alkyle.

9. Composés pesticides et herbicides, **caractérisés par** une teneur en au moins un composé de formule (I) suivant la revendication 1.

10. Utilisation de composés de formule (I) suivant la revendication 1 pour la lutte contre des parasites et des mauvaises herbes, excepté leur utilisation dans des procédés destinés au traitement du corps humain et animal.

11. Procédé de lutte contre des parasites et des mauvaises herbes, **caractérisé en ce qu'**on fait agir des composés de formule (I) suivant la revendication 1 sur des parasites et/ou sur leur milieu ou on les fait agir sur des mauvaises herbes et/ou sur leur milieu, excepté des procédés de traitement du corps humain ou animal.

12. Procédé de préparation de compositions pesticides et herbicides, **caractérisé en ce qu'**on mélange des composés de formule (I) suivant la revendication 1 avec des diluants et/ou des agents tensio-actifs.

13. Utilisation de composés de formule (I) suivant la revendication 1 pour la préparation de compositions pesticides et herbicides.

14. Composés de formule (XXII-a) 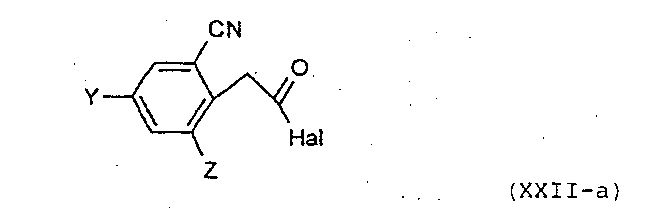 dans laquelle
Hal représente le chlore ou le brome et
Y et Z ont les définitions indiquées dans la revendication 1, mais ne représentent pas en même temps de l'hydrogène.
